# EUROPEAN PATENT SPECIFICATION

(11) **EP 4 043 886 B1**
(45) Date of publication and mention of the grant of the patent: **01.01.2025**
(21) Application number: 20911937.9
(22) Date of filing: 17.02.2020
(51) Int. Cl.: G01N 35/04, G01N 35/10, G01N 33/53, G01N 21/76, G01N 35/00

(54) **FULLY AUTOMATIC CHEMILUMINESCENT IMMUNE ANALYZER**
VOLLAUTOMATISCHER CHEMILUMINESZENZ-IMMUNANALYSATOR
ANALYSEUR D'IMMUNITÉ EN CHIMIOLUMINESCENCE ENTIÈREMENT AUTOMATIQUE

(30) Priority: 12.01.2020 CN 202010029112
(43) Date of publication of application: 17.08.2022
(73) Proprietor: Lansion Biotechnology Co., Ltd., Nanjing, Jiangsu 211122 (CN)
(72) Inventor: XU, Xingshang, Nanjing, Jiangsu 211122 (CN); CHEN, Jeffery, Nanjing, Jiangsu 211122 (CN); ZHAO, Daqiang, Nanjing, Jiangsu 211122 (CN)
(74) Representative: ZHAOffice SPRL
(86) International application number: PCT/CN2020/075519
(87) International publication number: WO 2021/138963

(56) References cited:
- EP-A1- 1 615 037
- WO-A1-2019/056234
- CN-A- 104 714 042
- CN-A- 106 918 715
- CN-A- 107 907 532
- CN-A- 108 061 810
- CN-A- 110 161 258
- CN-U- 208 092 069
- CN-U- 208 092 069
- CN-U- 211 402 409
- US-A1- 2009 042 281
- US-A1- 2020 217 865

## Description

### TECHNICAL FIELD

The present invention relates to the technical field of medical equipment, and in particular to a fully-automatic chemiluminescence immunoassay analyzer using chemiluminescence diagnosis technology.

### BACKGROUND

The mainstream chemiluminescence products in the market require serum or plasma as a test sample and cannot support direct, automated detection of a whole blood sample. Moreover, the long reporting time of the first sample and the long overall diagnostic time make it difficult to meet the requirements of the test turnaround time and result in a long overall waiting time. All of these exacerbate the pain of a patient and easily trigger resistance from family members or the patient himself/herself, thus being unconducive to subsequent treatment for the patient.

Chinese patent document No. CN106918715A discloses a chemiluminescence diagnostic device, which includes: a base, a sampling needle module, a magnetic-bead reagent pre-incubation module, an incubation and cleaning module, and a data analysis module. The base has a mounting face. The sampling needle module includes: a sampling mechanism rotatable with respect to the base, and a drive mechanism mounted on the base and used for driving the sampling mechanism to rotate, rise, and fall. The magnetic-bead reagent pre-incubation module includes a pre-incubation disk and a pre-incubation heating device for heating the pre-incubation disk, where the pre-incubation heating device is disposed on the bottom of the pre-incubation disk, and the pre-incubation disk is arranged about the axis of rotation of the sampling mechanism and located within a sampling region of the sampling mechanism. The incubation and cleaning module includes an incubation disk, an incubation heating device used for heating the incubation disk, a magnetic separation assembly, and a cleaning station assembly, where the incubation heating device is disposed on the bottom of the incubation disk; the magnetic separation assembly is sleeved on the outer edge of the incubation disk; the cleaning station assembly includes a drive assembly and a cleaning needle fixed on the drive assembly; and the cleaning needle is disposed above the incubation disk and can fit an inner cavity of an incubation reaction cup on the incubation disk in a working state. The data analysis module includes an industrial personal computer for data analysis and a touch display screen.

WO 2019/056234 A1 discloses an analyser according to the preamble of claim 1.

However, the chemiluminescence diagnostic device in the foregoing technical solution is not ideal in the specific use process.

### SUMMARY

The technical problem to be solved by the present invention is to provide a fully-automatic chemiluminescence immunoassay analyzer, where various modules can each operate independently, have flexible combinations and diverse functions, and can be adapted to different types of analytical instruments, and enable high detection efficiency, accurate and stable results, rapid sample analysis, and short chemiluminescence diagnosis time.

To solve the foregoing technical problems, the present invention adopts the following technical solution: The fully-automatic chemiluminescence immunoassay analyzer includes a base, where a reaction cup conveyance module, a robot-arm sample injection module, an incubation module, a manipulator and transit module, a magnetic-separation cleaning module, and a detection module are mutually independently integrated on the base, where:
the reaction cup conveyance module is used for conveying a reaction cup to a pre-determined position, such that the robot-arm sample injection module can add a reaction reagent and a sample separately into the reaction cup;
the manipulator and transit module carries the reaction cup into the incubation module, to incubate the reaction cup for a constant-temperature reaction; and
the reaction cup in the incubation module is carried by the manipulator and transit module to the magnetic-separation cleaning module for magnetic adsorption cleaning; and the cleaned reaction cup is carried by the manipulator and transit module to the detection module for detection.

By use of the foregoing technical solution, various module parts are all integrated on the base to form an equipment set, realizing structure integration and streamlined operation, shortening the test time, and achieving a good use effect. The modules can operate independently, have flexible combinations and diverse functions, and can be adapted to different types of analytical instruments, and enable high detection efficiency and accurate and stable results. The manipulator and transit module is used for transit of the reaction cup in the detection process, and enables the detection time to be shortened by cooperation with the manipulators for carrying. The manipulators can make horizontal, rotary, and up-and-down motions, and have a retractable gripping jaw; and thus can be used to realize high-efficiency detection. A plurality of manipulators and a plurality of transit devices may be disposed as required, to link various mutually independent functional parts of the modules. Further, the instrument may be equipped with detection modules and process modules of various detection platforms, which cooperate with the manipulator and transit module to realize diverse detection in a table-top production line manner.

The manipulator and transit module includes a first manipulator, a second manipulator, and a transit device; the first manipulator is used for carrying the reaction cup in the incubation module to the magnetic-separation cleaning module for cleaning, and carrying it again to the transit device; and the second manipulator is used for carrying the reaction cup on the transit device to the detection module for detection.

The transit device is used for transit of the reaction cup in the detection process, and enables the detection time to be shortened in cooperation with the manipulators.

The first manipulator and the second manipulator both have a gripping jaw used for carrying the reaction cup; the gripping jaw is disposed on a gripping jaw retractable rack and the gripping jaw retractable rack is controlled by a retractable motion motor; a slider is connected to the retractable motion motor, and is driven by an up-and-down motion motor to drive the gripping jaw to move up and down; and a rotation shaft is further connected to the retractable motion motor and is driven by a rotary motion motor to rotate, thus driving the gripping jaw to rotate.

Capable of making rotary, up-and-down, and retractable motions, the first manipulator and the second manipulator can perform movement in three dimensions, thus flexibly controlling the gripping jaws to carry the reaction cup. The up-and-down motion is controlled by the up-and-down motion motor, thus realizing an up-and-down motion along the rotation shaft. When started, the rotary motion motor drives the rotation shaft to rotate, and the gripping jaw can rotate accordingly. The retractable motion motor controls extension or retraction of the gripping jaw, and drives the gripping jaw retractable rack to make a retractable motion.

The first manipulator is further provided with an operating base; a first-manipulator horizontal motion screw rod is sleeved on the operating base, and is driven by a first-manipulator horizontal motion motor, such that the first manipulator can move back and forth along a first-manipulator horizontal motion guide rail.

The first manipulator moves horizontally by means of the operating base; and further moves back and forth along the first-manipulator horizontal motion guide rail with the cooperation between the first-manipulator horizontal motion screw rod and the first-manipulator horizontal motion guide rail. Thus, the first manipulator can make movement in four dimensions.

Preferably, the transit device includes a transit base which is provided with several cup hole sites for accommodating the reaction cups, and a transit rotary motor is disposed below the transit base and used for driving the transit base to make a corresponding rotary motion.

The transit device is used for transit of the reaction cup in the detection process, and enables the detection time to be shortened in cooperation with the manipulators. The first manipulator carries the incubated reaction cup to the magnetic-separation cleaning module for cleaning and then carries the cleaned reaction cup to the transit device; and the second manipulator carries the reaction cup from the transit device to the detection module for detection. The transit rotary motor drives the transit base to make a corresponding rotary motion, to adjust the cup hole site of the reaction cup on the transit base and the orientation of the reaction cup in the cup hole site, thereby shortening the transit time in cooperation with the gripping jaws of the first and second manipulators for carrying.

Preferably, the robot-arm sample injection module includes a first sampling device disposed with a sampling needle assembly and a second sampling device disposed with a pipette tip assembly, where the first sampling device is used for drawing the reaction reagent and adding it to the reaction cup, and the second sampling device is used for drawing the sample and adding it to the reaction cup; and the robot-arm sample injection module further includes a transverse motion assembly, a longitudinal motion assembly, and an up-and-down motion assembly, where the transverse motion assembly drives the first sampling device and the second sampling device separately to make a transverse motion, the longitudinal motion assembly drives the first sampling device and the second sampling device separately to make a longitudinal motion, and the up-and-down motion assembly drives the first sampling device and the second sampling device separately to make an up-and-down motion.

The pipette tip assembly draws the sample and then adds it to the reaction cup; and the sampling needle assembly draws the reaction reagent and adds it to the reaction cup to react with the sample. The transverse motion assembly, the longitudinal motion assembly, and the up-and-down motion assembly are used to drive the first sampling device and the second sampling device to make movements in three dimensions: transverse, longitudinal, and up-and-down directions, thus flexibly controlling the first sampling device and the second sampling device, realizing a high level of automation, and greatly reducing the addition time of the sample and the reaction reagent.

Preferably, the transverse motion assembly includes a first transverse motion motor which drives a first transmission shaft to rotate, and drives the first sampling device via the first transmission shaft to move transversely on a first transverse motion shaft; and the transverse motion assembly further includes a second transverse motion motor which drives a second transmission shaft to rotate and drives the second sampling device via the second transmission shaft to move transversely on a second transverse motion shaft.

The transverse motion assembly uses a first transverse motion motor and a second transverse motion motor to separately control the transverse motion of the first sampling device and the second sampling device, without mutual interference, thus realizing a high level of automation, a rapid response, and flexible movement.

Preferably, the longitudinal motion assembly includes a first longitudinal motion motor which drives the first sampling device to move longitudinally along a first longitudinal motion shaft and a second longitudinal motion shaft; and the longitudinal motion assembly further includes a second longitudinal motion motor which drives the second sampling device to move longitudinally along a third longitudinal motion shaft and a fourth longitudinal motion shaft.

The longitudinal motion assembly uses a first longitudinal motion motor and a second longitudinal motion motor to separately control the longitudinal motion of the first sampling device and the second sampling device, without mutual interference, thus realizing a high level of automation, a rapid response, and flexible movement.

Preferably, the up-and-down motion assembly includes a first up-and-down motion motor, the sampling needle assembly and a first up-and-down motion slider are connected, the first up-and-down motion slider is sleeved on a first up-and-down motion screw rod, the first up-and-down motion motor drives the first up-and-down motion screw rod to move, and the first up-and-down motion slider on the first up-and-down motion screw rod drives the sampling needle assembly to move up and down along a first guide rail; the up-and-down motion assembly further includes a second up-and-down motion motor, the pipette tip assembly and a second up-and-down motion slider are connected, the second up-and-down motion slider is sleeved on a second up-and-down motion screw rod, the second up-and-down motion motor drives the second up-and-down motion screw rod to move, and the second up-and-down motion slider on the second up-and-down motion screw rod drives the pipette tip assembly to move up and down along a second guide rail.

The up-and-down motion assembly uses a first up-and-down motion motor and a second up-and-down motion motor to separately control the up-and-down motion of the first sampling device and the second sampling device, without mutual interference, thus realizing a high level of automation, a rapid response, and flexible movement.

Preferably, the reaction cup conveyance module includes a reaction cup conveyance chamber, and the reaction cup falling from the reaction cup conveyance chamber is adjusted in posture by means of a reaction cup flipper; and the reaction cup conveyance module further includes a reaction cup carrier which pushes the reaction cup after posture adjustment to a designated position, where a moving track of the reaction cup carrier is below the incubation module.

After the reaction cup carrier pushes the reaction cup after posture adjustment to the designated position, the sample and the reaction reagent are added to the reaction cup (by the pipette tip assembly and the sampling needle assembly respectively). Further, structurally, the moving track of the reaction cup carrier is below the incubation module, and thus the reaction cup can be carried by the manipulator and transit module into the incubation module, for an incubation reaction.

Preferably, the reaction cup slides in along a reaction cup cassette holder, and a reaction cup transfer bracket is located on the bottom portion of the reaction cup conveyance chamber; a reaction cup transfer groove that fits the reaction cup cassette holder is disposed on the reaction cup transfer bracket, and the reaction cup transfer bracket moves back and forth with the synergy of a cup transfer fitting groove; and the reaction cup in the reaction cup conveyance chamber enters the reaction cup transfer groove and then falls into the reaction cup flipper.

The reaction cup transfer groove that fits the reaction cup cassette holder is disposed, and the reaction cup transfer groove has a hollow-carved design at a position corresponding to the reaction cup cassette holder. When moving to this position, the reaction cup goes into the reaction cup transfer groove. The reaction cup transfer bracket moves back and forth with the synergy of the cup transfer fitting groove. When the reaction cup transfer bracket moves forwards, the reaction cup falls to a place below the reaction cup transfer bracket that is hollow-carved, and therefore, the reaction cup falls into the reaction cup flipper from this place, for posture adjustment.

Preferably, the robot-arm sample injection module adds the reaction reagent and the sample separately to the reaction cup which is pushed by the reaction cup carrier to the designated position, and then the manipulator and transit module carries the reaction cup into an incubation base of the incubation module; and the incubation base is secured on an incubation base vertical panel, where an incubation sheet is disposed on the bottom of the incubation base and insulating sheets are respectively disposed at the two sides of the incubation base.

Preferably, the magnetic-separation cleaning module includes a magnetic-separation cleaning needle, and a rotatable magnetic-separation seat is disposed below the magnetic-separation cleaning needle, the magnetic-separation seat having at least one reaction cup hole. The magnetic-separation cleaning module further includes a magnetic cleaning pool, where the magnetic cleaning pool includes a cleaning solution path and a cleaning needle liquid discharge path. A cleaning solution enters through a cleaning solution inlet in the cleaning solution path and then goes into the reaction cup through a cleaning solution pointed outlet for cleaning; and the magnetic-separation cleaning needle is used for discharging the waste liquid in the reaction cup after cleaning through the cleaning needle liquid discharge path.

The magnetic-separation cleaning module performs magnetic-separation cleaning for a detection target, to clean off unreacted substances and impurities, such that the cleaned target waits for entering the detection module for detection. The reaction cup after the incubation reaction by the incubation module is placed in the reaction cup hole on the magnetic-separation seat, and multiple reaction cup holes may be provided. It should be noted that, while flowing through the cleaning solution pointed outlet, the cleaning solution can also clean the cleaning needle. The cleaning needle is used for drawing up and discharging the waste liquid in the reaction cup after cleaning, and the cleaning process includes 3 to 5 liquid introduction and discharge operations. The magnetic-separation seat may be designed to a rotatable seat plate, such that the reaction cups placed thereon can be successively cleaned by rotation.

Preferably, an up-and-down motion of the magnetic-separation cleaning needle is controlled by a magnetic-cleaning lifting motor; the magnetic-separation cleaning module is further disposed with a lifting limit structure; the lifting limit structure includes a magnetic-cleaning lifting upper plate and a magnetic-cleaning lifting lower plate, where a limit optocoupler, a cleaning needle lifting stopper, a lifting screw rod, and a guide rod are disposed between the magnetic-cleaning lifting upper plate and the magnetic-cleaning lifting lower plate; a slider is slidably connected on the lifting screw rod, the magnetic-cleaning lifting motor drives the lifting screw rod to rotate, the slider is connected to the guide rod and the magnetic-separation cleaning needle, and the magnetic-separation cleaning needle is driven by the slider to move up and down along the guide rod.

Preferably, the magnetic-separation seat is driven by a magnetic-separation seat rotary motor to rotate, and a rotation limit structure is disposed below the magnetic-separation seat rotary motor; and a magnet is disposed inside the magnetic-separation seat.

The magnet is disposed inside the magnetic-separation seat and used for adsorbing an target substance to be tested in the sample.

Preferably, the detection module includes a detection chamber, a PMT, and a photon counter; the reaction cup is carried by the manipulator and transit module to a reaction cup detection seat of the detection chamber; a first trigger solution and a second trigger solution are successively added to the reaction cup respectively through a first trigger solution pipeline and a second trigger solution pipeline, and after reaction, the waste liquid is discharged through a waste liquid discharge pipeline of the detection module; and the PMT and the photon counter are used to collect and convert optical signals into electrical signals, to finally obtain a detection result through analysis.

Preferably, the detection module further includes a motor for trigger solution refilling and waste liquid tube lifting motions, which can drive simple screw rods to move up and down; the simple screw rods are slidably connected to detection module guide rods, to drive the first trigger solution pipeline, the second trigger solution pipeline, and the waste liquid discharge pipeline to respectively move up and down along the detection module guide rods; the detection module guide rods are connected to a guide rod fixing block, and are further respectively connected to the first trigger solution pipeline, the second trigger solution pipeline, and the waste liquid discharge pipeline through a connecting piece; and a limit stopper is disposed on the connecting piece and cooperates with an optocoupler, for limiting an upward motion.

Preferably, when the reaction cup is carried by the manipulator and transit module into the reaction cup detection seat of the detection chamber, a detection chamber baffle moves so that a reaction cup transfer hole and a baffle through hole on the detection chamber baffle coincide, and then the reaction cup is put in. A reaction cup lifting control motor drives a motor rotation connecting pillar to rotate, to drive a lift rod carrier connecting block to move up and down with the synergy of a lift rod movement fitting groove, where a lift rod carrier is fixedly connected on the lift rod carrier connecting block; and the lift rod carrier is connected to a reaction cup lift rod, and the reaction cup lift rod moves up and down to take the reaction cup into the reaction cup detection position.

Preferably, the detection chamber baffle is mounted below a detection chamber top cover; the baffle motion motor drives a baffle motor rotation connecting pillar to rotate, such that the detection chamber baffle moves back and forth with the synergy of a baffle motion fitting groove; and accordingly, the baffle through hole on the detection chamber baffle and the reaction cup transfer hole coincide or are misaligned, to open or close the detection chamber.

By the foregoing structural design, the detection chamber is ensured to be a dark room, thus guaranteeing a light avoidance reaction.

Preferably, the first trigger solution pipeline, the second trigger solution pipeline, and the waste liquid discharge pipeline all run through the detection chamber top cover; the trigger solution is added to the reaction cup in the reaction cup detection seat; and the waste liquid discharge pipeline is relatively long, and is able to stick into the liquid level in the reaction cup for liquid drawing and discharge.

The motion principle of the fully-automatic chemiluminescence immunoassay analyzer of the present invention is as follows: The reaction cup enters the instrument by the reaction cup conveyance module, and the reaction cup carrier takes it to a designated position; and then the robot-arm sample injection module adds the sample and the reaction reagent separately into the reaction cup. After carrying the reaction cup to the incubation module for an incubation reaction, the first manipulator carries the reaction cup to the magnetic-separation cleaning module. The magnetic-separation cleaning needle draws and discharges the waste liquid that has not been reacted and bound in the reaction cup, and a cleaning solution is added to the reaction cup for cleaning; and then the magnetic-separation cleaning needle draws and discharges the waste liquid once again. This process is repeated for 3 to 5 times. After cleaning completion, the first manipulator carries the reaction cup to the transit device in the manipulator and transit module; and the second manipulator carries the reaction cup to the detection module and an enhancement solution is added in, to carry out a light avoidance reaction. Then the PMT and the photon counter are used for detection. The first manipulator can make horizontal, rotary, and up-and-down motions, and has a retractable gripping jaw, thus cooperating with the second manipulator and the transit device to implement high-efficiency detection. A plurality of manipulators and a plurality of transit devices may be disposed as required. Further, the instrument may be equipped with detection modules and process modules of various detection platforms, which cooperate with the manipulators and the transit devices to realize diverse detection in a table-top production line manner.

### BRIEF DESCRIPTION OF THE DRAWINGS

Detailed description is further made below with reference to the accompanying drawings and specific implementations of the present invention:
FIG. 1 is a schematic structural diagram of a fully-automatic chemiluminescence immunoassay analyzer of the present invention;
FIG 2 is a top view of the fully-automatic chemiluminescence immunoassay analyzer in FIG 1 from which a robot-arm sample injection module is removed;
FIG 3 is a three-dimensional diagram of the fully-automatic chemiluminescence immunoassay analyzer in FIG 1 from which a robot-arm sample injection module is removed;
FIG 4 is a three-dimensional diagram of a manipulator and transit module;
FIG 5 is a three-dimensional structural diagram of a first manipulator in FIG. 4;
FIG 6 is a sectional structural diagram of a retractable motion motor of the first manipulator in FIG 5;
FIG. 7 is a three-dimensional structural diagram of a second manipulator in FIG 4;
FIG. 8 is a sectional structural diagram of a retractable motion motor of the second manipulator in FIG. 7;
FIG. 9 is a first three-dimensional structural diagram of a robot-arm sample injection module;
FIG. 10 is a second three-dimensional structural diagram of the robot-arm sample injection module;
FIG. 11 is a schematic three-dimensional structural diagram of a spring clamp in FIGs. 9 and 10;
FIG. 12a is a first three-dimensional structural diagram of a longitudinal motion assembly of a first sampling device of the robot-arm sample injection module;
FIG. 12b is a second three-dimensional structural diagram of the longitudinal motion assembly of the first sampling device of the robot-arm sample injection module;
FIG. 13 is a three-dimensional structural diagram of a longitudinal motion assembly of a second sampling device of the robot-arm sample injection module;
FIG. 14 is a three-dimensional structural diagram of an up-and-down motion assembly of the first sampling device of the robot-arm sample injection module;
FIG. 15 is a three-dimensional structural diagram of an up-and-down motion assembly of the second sampling device of the robot-arm sample injection module;
FIG. 16 is a schematic limited structural diagram of a pipette tip assembly of the second sampling device of the robot-arm sample injection module;
FIG. 17 is a schematic limited structural diagram of a sampling needle assembly of the first sampling device of the robot-arm sample injection module;
FIG. 18 is a schematic three-dimensional structural diagram of the pipette tip assembly of the second sampling device of the robot-arm sample injection module;
FIG. 19 is a schematic three-dimensional structural diagram of a sampling needle assembly of the first sampling device of the robot-arm sample injection module;
FIG. 20 is a schematic structural diagram of a pipette tip holder in the robot-arm sample injection module;
FIG. 21 is a schematic structural diagram of a pipette tip holder motion assembly in FIG 20;
FIG. 22 is a schematic three-dimensional structural diagram of a sample tube bin assembly in the robot-arm sample injection module;
FIG. 23 is a schematic structural diagram of a sample tube cassette in the sample tube bin assembly in FIG 22;
FIG. 24 is a schematic structural diagram of a kit bin in the robot-arm sample injection module;
FIG. 25 is a schematic structural diagram of a kit holder in the kit bin in FIG 24;
FIG 26 is a schematic structural diagram of a cover plate motion assembly in the robot-arm sample injection module;
FIG 27 is a schematic structural diagram of a sampling needle cleaning pool in the kit bin in FIG 24;
FIG 28 is a schematic three-dimensional structural diagram of a reaction cup conveyance module;
FIG 29 is a schematic three-dimensional structural diagram of a reaction cup conveyance chamber assembly in the reaction cup conveyance module;
FIG 30 is a three-dimensional bottom view of a reaction cup conveyance chamber in the reaction cup conveyance module;
FIG 31 is a schematic three-dimensional structural diagram of a reaction cup transfer bracket assembly in the reaction cup conveyance module;
FIG 32 is a schematic three-dimensional structural diagram of the reaction cup conveyance module equipped with an incubation module;
FIG. 33 is a schematic three-dimensional structural diagram of the incubation module;
FIG 34 is a first three-dimensional diagram of a magnetic-separation cleaning module;
FIG 35 is a partial sectional diagram of a cleaning solution path shown in the magnetic-separation cleaning module in FIG. 34;
FIG 36 is a second three-dimensional diagram of the magnetic-separation cleaning module;
FIG 37 is a sectional diagram of a magnetic-separation cleaning device;
FIG 38 is a three-dimensional diagram of the magnetic-separation cleaning device;
FIG 39 is a partial sectional three-dimensional diagram of the magnetic-separation cleaning device in the magnetic-separation cleaning module;
FIG 40 is a three-dimensional structural diagram of a detection module;
FIG 41 is a three-dimensional structural diagram of the detection module from which a detection chamber housing is removed;
FIG 42 is a partial structural diagram of a first trigger solution pipeline, a second trigger solution pipeline, and a waste liquid discharge pipeline shown in the detection module;
FIG 43 is a diagram showing a mounting relationship regarding a detection chamber baffle in the detection module from which a detection chamber top cover is removed;
FIG 44 is a schematic diagram of a motion status of a reaction cup lift rod in the detection module;
FIG 45 is a schematic structural diagram of a detection chamber baffle in the detection module;
FIG 46 is a schematic structural bottom view of the detection chamber baffle in the detection module;
FIG 47 is a sectional diagram of the detection module; and
FIG 48 is a schematic partial structural diagram of a liquid path module of the fully-automatic chemiluminescence immunoassay analyzer in FIG 1.

Meanings of numerals: 1. Base; 2. Reaction cup conveyance module; 201. Reaction cup conveyance chamber; 202. Reaction cup flipper; 203. Reaction cup carrier; 20301. guide rail; 20302. Reaction cup pusher; 20303. Flipper pivot; 204. Reaction cup cassette holder; 205. Reaction cup transfer bracket; 20501. Reaction cup transfer groove; 20502. Cup transfer fitting groove; 206. Reaction cup transfer motor; 207. Motor rotation connecting block; 208. Reaction cup conveyance motor; 209. Screw rod; 210. Reaction cup carrier slider; 3. Robot-arm sample injection module; 301. First sampling device; 30101. Sampling needle assembly; 30102. Sampling needle; 30103. Needle point protection magnet; 30104. First capacitance probe; 30105. Second PCB board; 30106. First elastic copper sheet; 30107. Sampling needle fitting joint; 30108. First spring; 30109. First connection block; 30110. Magnetic field sensing element; 30111. Sampling needle liquid contact; 30112. First PCB board; 30113. Pressure sensor; 30114. First longitudinal motion connection block; 302. Second sampling device; 30201. Pipette tip assembly; 30202. Pipette tip; 30203. Second capacitance probe; 30204. Fourth PCB board; 30205. Second elastic copper sheet; 30206. Pipette tip engaging portion; 30207. Second spring; 30208. Second connection block; 30209. Pipette tip pickup confirmation magnet; 30210. Magnetic field sensing element; 30211. Pipette tip liquid contact; 30212. Second longitudinal motion connection block; 30213. Third PCB board; 30214. Pressure sensor; 303. Transverse motion assembly; 30301. First transverse motion motor; 30302. First transmission shaft; 30303. First transverse motion shaft; 30304. Second transverse motion motor; 30305. Second transmission shaft; 30306. Second transverse motion shaft; 30307. First synchronous pulley; 30308. Second synchronous pulley; 30309. Fourth synchronous pulley; 30310. Seventh synchronous pulley; 30311. Ninth synchronous pulley; 30312. First synchronous belt; 30313. Third synchronous belt; 30314. Spring clamp; 30315. Spring clamp fixing block; 30316. Sixth synchronous pulley; 30317. Eighth synchronous pulley; 30318. Tenth synchronous pulley; 30319. Third synchronous pulley; 30320. Fifth synchronous pulley; 30321. Second synchronous belt; 30322. Fourth synchronous belt; 30323. Spring clamp; 30324. Spring clamp fixing block; 30325. Spring; 304. Longitudinal motion assembly; 30401. First longitudinal motion motor; 30402. First longitudinal motion shaft; 30403. Second longitudinal motion shaft; 30404. Second longitudinal motion motor; 30405. Third longitudinal motion shaft; 30406. Fourth longitudinal motion shaft; 30407. First driving pulley; 30408. Fifth belt; 30409. First driven pulley; 30410. Second driving pulley; 30411. Sixth belt; 30412. Second driven pulley; 305. Up-and-down motion assembly; 30501. First up-and-down motion motor; 30502. First up-and-down motion slider; 30503. First up-and-down motion screw rod; 30504. First guide rail; 30505. Second up-and-down motion motor; 30506. Second up-and-down motion slider; 30507. Second up-and-down motion screw rod; 30508. Second guide rail; 30509. Second limit magnet; 30510. Second limit magnetic field sensing element; 30511. First limit magnet; 30512. First limit magnetic field sensing element; 306. Pipette tip holder; 30601. Pipette tip cassette rail; 30602. Pipette tip holder rail; 307. Sample tube bin assembly; 30701. Sample tube rest; 30702. Sample tube cassette; 30703. Elastic steel ball part; 30704. Sample tube; 30705. Steel ball fitting groove; 30706. Handle; 30707. Projection; 30708. Trigger solution storage box; 308. Kit bin; 30801. Cover plate motion assembly; 30802. Kit assembly; 30803. Kit holder connecting block; 30804. Kit mixing motor; 30805. Motor rotation connecting block; 30806. Connecting rod; 30807. Mixing support shaft; 30808. Kit holder; 30809. Kit; 30810. Elastic steel ball part; 30811. Steel ball fitting groove; 30812. Projection; 30813. Cover plate; 30814. Motor rotation connecting block; 30815. Cover plate movement fitting groove; 30816. Cover plate motion motor; 30817. Sampling needle cleaning pool; 30818. Sampling isolation plate; 30819. Cleaning pool; 30820. Cleaning pool spare chamber; 30821. Liquid discharge hole; 30822. Handle; 30823. Projection; 309. Waste bin; 3010. Trigger solution bin; 4. Incubation module; 401. Incubation base; 402. Incubation base vertical panel; 403. Incubation sheet; 404. Insulating sheet; 5. Manipulator and transit module; 501. First manipulator; 50101. Gripping jaw; 50102. Gripping jaw retractable rack; 50103. Retractable motion motor; 50104. Horizontal motion assembly; 50105. Up-and-down motion motor; 50106. Rotation shaft; 50107. Rotary motion motor; 50108. Operating base; 50109. First-manipulator horizontal motion screw rod; 50110. First-manipulator horizontal motion motor; 50111. First-manipulator horizontal motion guide rail; 50112. First-manipulator horizontal motion driving pulley; 50113. First-manipulator horizontal motion driven pulley; 50114. Up-and-down motion screw rod; 50115. Up-and-down motion slider; 50116. Rotary motion driving pulley; 50117. Rotary motion driven pulley; 50118. Retractable motion motor gear; 502. Second manipulator; 50201. Gripping jaw; 50202. Gripping jaw retractable rack; 50203. Retractable motion motor; 50204. Up-and-down motion slider; 50205. Up-and-down motion motor; 50206. Rotation shaft; 50207. Rotary motion motor; 50208. Up-and-down motion screw rod; 50209. Rotary motion driving pulley; 50210. Rotary motion driven pulley; 50211. Retractable motion motor gear; 503. Transit device; 50301. Transit base; 50302. Cup hole site; 50303. Transit rotary motor; 6. Magnetic-separation cleaning module; 601. Magnetic-separation cleaning needle; 602. Magnetic-separation seat; 60201. Reaction cup hole; 60202. Spare reaction cup hole; 60203. Magnet; 603. Magnetic cleaning pool; 60301. Cleaning solution path; 60302. Cleaning needle liquid discharge path; 604. Cleaning solution inlet; 605. Cleaning solution pointed outlet; 606. Magnetic-cleaning lifting motor; 607. Lifting limit structure; 60701. Magnetic-cleaning lifting upper plate; 60702. Magnetic-cleaning lifting lower plate; 60703. Limit Optocoupler; 60704. Cleaning needle lifting stopper; 60705. Lifting screw rod; 60706. Guide rod; 60707. Slider; 60708. Magnetic-cleaning lifting motor; 608. Magnetic-separation seat rotary motor; 609. Rotation limit structure; 610. Diaphragm pump; 7. Detection module; 701. Detection chamber; 70101. Detection chamber top cover; 702-PMT; 703. Photon counter; 704. Reaction cup detection seat; 705. First trigger solution pipeline; 706. Second trigger solution pipeline; 707. Waste liquid discharge pipeline; 708. Motor for trigger solution refilling and waste liquid tube lifting motions; 70801. Driving pulley; 70802. Driven pulley; 70803. Belt; 709. Simple screw rod; 710. Detection module guide rod; 711. Guide rod fixing block; 712. Connecting piece; 713. Limit stopper; 714. Optocoupler; 715. Detection chamber baffle; 71501. Baffle through hole; 716. Reaction cup transfer hole; 717. Reaction cup lifting control motor; 718. Motor rotation connecting pillar; 719. Lift rod carrier connecting block; 720. Lift rod movement fitting groove; 721. Lift rod carrier; 722. Reaction cup lift rod; 723. Reaction cup detection position; 724. Baffle motion motor; 725. Baffle motor rotation connecting pillar; 726. Baffle motion fitting groove; 727. Reaction cup detection seat rotary motor; 72701. Rotation driving pulley; 72702. Rotation driven pulley; 8. Liquid path module; 801. Cleaning solution pool entrance; 802. Cleaning solution pool; 803. Cleaning solution pool branch outlet; 804. Waste reservoir branch inlet; 805. Waste reservoir; 806. Waste reservoir exit; 807. Piston pump; 808. Waste reservoir entrance; 809. Cleaning solution pool exit; 811. Solenoid valve; 810. Trigger solution dosing pump; 9. Reaction cup

### DETAILED DESCRIPTION OF THE INVENTION

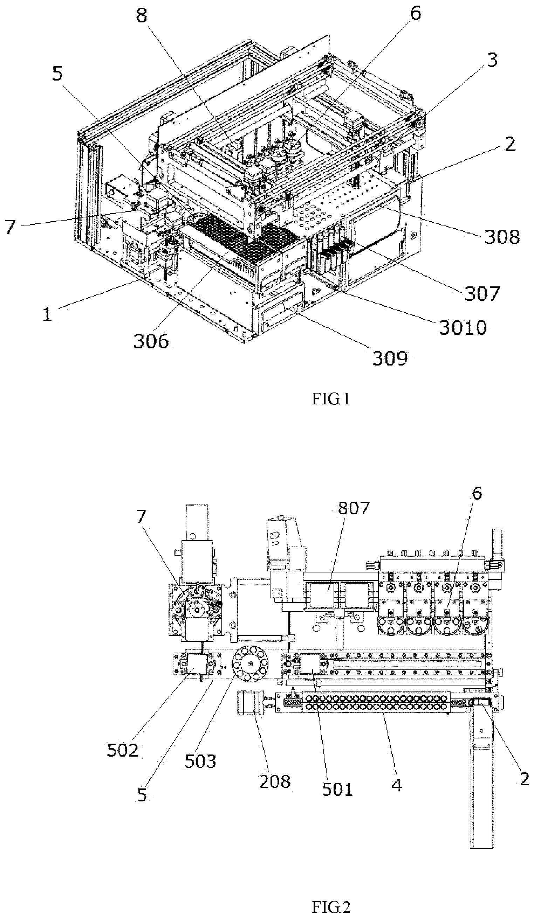

As shown in FIG 1, a fully-automatic chemiluminescence immunoassay analyzer in this embodiment includes a base 1, where a reaction cup conveyance module 2, a robot-arm sample injection module 3, an incubation module 4, a manipulator and transit module 5, a magnetic-separation cleaning module 6, and a detection module 7 are mutually independently integrated on the base 1. The reaction cup conveyance module 2 is used for conveying a reaction cup 9 to a pre-determined position, such that the robot-arm sample injection module 3 can add a sample and a reaction reagent separately into the reaction cup 9. The manipulator and transit module 5 carries the reaction cup 9 into the incubation module 4 (as shown in FIG 2), to incubate the reaction cup 9 for a constant-temperature reaction. The reaction cup 9 in the incubation module 4 is carried by the manipulator and transit module 5 to the magnetic-separation cleaning module 6 for magnetic-adsorption cleaning; and the cleaned reaction cup 9 is carried by the manipulator and transit module 5 to the detection module 7 for detection.

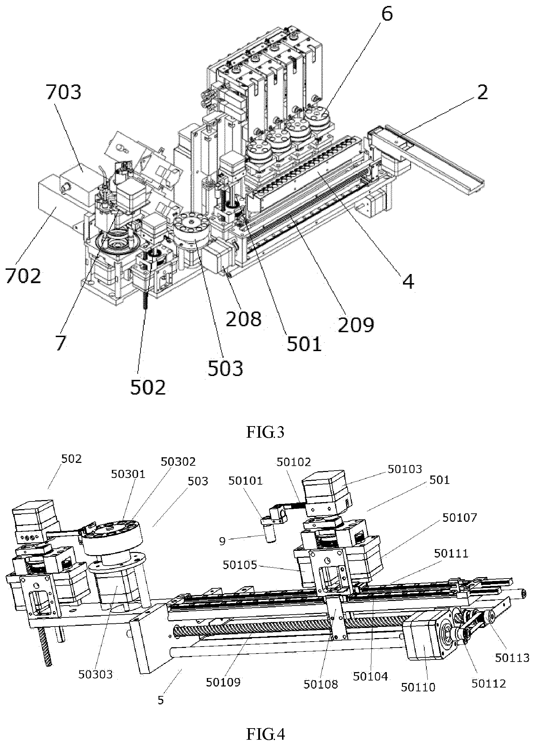

As shown in FIG 4, the manipulator and transit module 5 includes a first manipulator 501, a second manipulator 502, and a transit device 503. The first manipulator 501 is used for carrying the reaction cup 9 in the incubation module 4 to the magnetic-separation cleaning module 6 for cleaning, and carrying it again to the transit device 503. The second manipulator 502 is used for carrying the reaction cup 9 on the transit device 503 to the detection module 7 for detection.

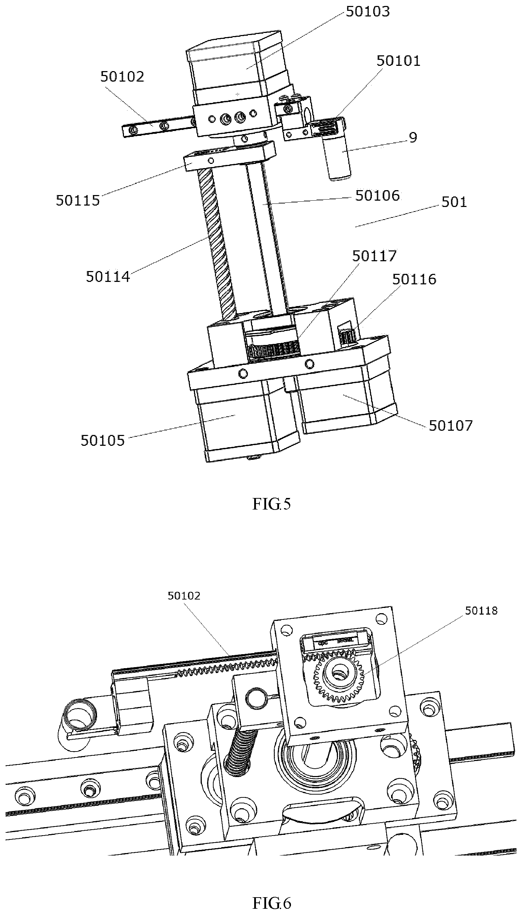

As shown in FIGs. 4 and 5, the first manipulator 501 includes a gripping jaw 50101 used for carrying the reaction cup 9, where the gripping jaw 50101 is disposed on a gripping jaw retractable rack 50102 and the gripping jaw retractable rack 50102 is controlled by a retractable motion motor 50103. An up-and-down motion slider 50115 is connected to the retractable motion motor 50103, and is driven by an up-and-down motion motor 50105 to drive the gripping jaw 50101 to move up and down. A rotation shaft 50106 is further connected to the retractable motion motor 50103, and is driven by a rotary motion motor 50107 to rotate, thus driving the gripping jaw 50101 to rotate.

The first manipulator 501 is further provided with an operating base 50108. As shown in FIG 4, a first-manipulator horizontal motion screw rod 50109 is sleeved on the operating base 50108, and is driven by a first-manipulator horizontal motion motor 50110, such that the first manipulator 501 can move back and forth along a first-manipulator horizontal motion guide rail 50111. These components constitute a horizontal motion assembly 50104 of the first manipulator 501.

In this embodiment, the first manipulator 501 is responsible for carrying the reaction cup 9 incubated by the incubation module 4 to the magnetic-separation cleaning module 6 for cleaning; and further carrying the cleaned reaction cup to the transit device 503. The second manipulator 502 is responsible for carrying the reaction cup 9 from the transit device 503 to the detection module 7 for detection.

The first manipulator 501 can make horizontal, rotary, up-and-down, and retractable motions, to flexibly control the gripping jaw 50101 to carry the reaction cup 9. The horizontal motion is realized by cooperation between the first-manipulator horizontal motion motor 50110, the first-manipulator horizontal motion driving pulley 50112, the first-manipulator horizontal motion driven pulley 50113, the first-manipulator horizontal motion screw rod 50109, and the first-manipulator horizontal motion guide rail 50111. The operating base 50108 drives the first manipulator 501 to move horizontally. The up-and-down motion is controlled by an up-and-down motion motor 50105 which drives an up-and-down motion screw rod 50114 to rotate. The up-and-down motion screw rod 50114 is slidably connected to an up-and-down motion slider 50115; and the up-and-down motion slider 50115 is slidably connected to a rotation shaft 50106 and is fixedly connected to a gripping jaw retractable motion assembly such as a retractable motion motor 50103 and the like. Therefore, driven by the up-and-down motion slider 50115, the gripping jaw 50101 can move up and down along the rotation shaft 50106. After a rotary motion motor 50107 is started, a rotary motion driving pulley 50116 drives a rotary motion driven pulley 50117 to rotate, to drive the rotation shaft 50106 to rotate. Because the rotation shaft 50106 is fixedly connected to the gripping jaw retractable motion assembly such as the retractable motion motor 50103 and the like, the gripping jaw 50101 can rotate. The retractable motion motor 50103 controls extension or retraction of the gripping jaw 50101, and a retractable motion motor gear 50118 is used to drive a gripping jaw retractable rack 50102 to make a retractable motion. Therefore, the gripping jaw 50101 can make a retractable motion, as shown in FIG 6.

The transit device 503 is used for transit of the reaction cup in a detection process, and includes a transit base 50301 which is provided with several cup hole sites 50302 for accommodating the reaction cups. In this embodiment, as shown in FIG 4, there are ten cup hole sites. A transit rotary motor 50303 is disposed below the transit base 50301, and used for driving the transit base 50301 to make a corresponding rotary motion.

The detection time can be shortened by cooperation between the first and second manipulators 501 and 502. The second manipulator 502 has the same structure as the first manipulator 501, but does not have the horizontal motion assembly 50104; however, a horizontal motion assembly can be disposed if required.

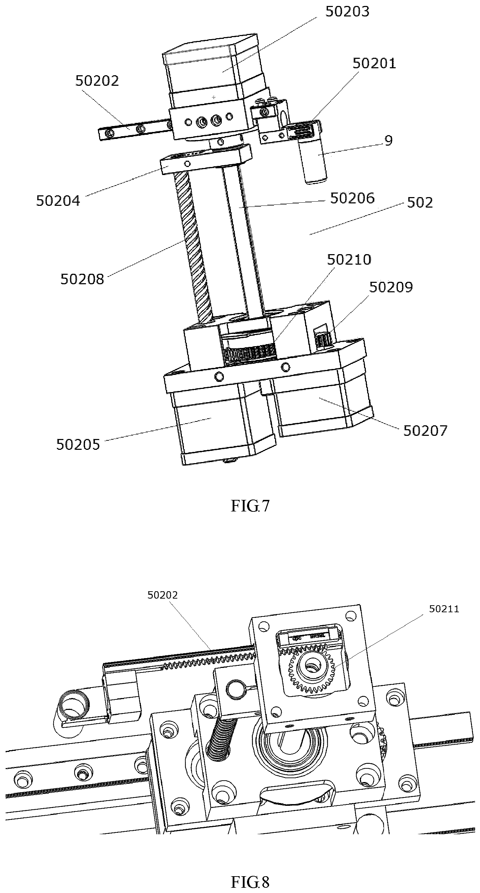

Specifically, as shown in FIG 7, the second manipulator 502 includes a gripping jaw 50201 which is used for carrying the reaction cup 9 and disposed on a gripping jaw retractable rack 50202, where the gripping jaw retractable rack 50202 is controlled by a retractable motion motor 50203. An up-and-down motion slider 50204 is connected to the retractable motion motor 50203 and is driven by an up-and-down motion motor 50205, thus driving the gripping jaw 50201 to move up and down. A rotation shaft 50206 is further connected to the retractable motion motor 50203 and is driven by a rotary motion motor 50207 to rotate, thus driving the gripping jaw 50201 to rotate.

The second manipulator 502 can make rotary, up-and-down, and retractable motions, to flexibly control the gripping jaw 50201 to carry the reaction cup 9. The up-and-down motion is controlled by the up-and-down motion motor 50205 which drives an up-and-down motion screw rod 50208 to rotate. The up-and-down motion screw rod 50208 is slidably connected to an up-and-down motion slider 50204; and the up-and-down motion slider 50204 is slidably connected to the rotation shaft 50206 and is fixedly connected to a gripping jaw retractable motion assembly such as the retractable motion motor 50203 and the like. Therefore, driven by the up-and-down motion slider 50204, the gripping jaw 50201 can move up and down along the rotation shaft 50206. After a rotary motion motor 50207 is started, a rotary motion driving pulley 50209 drives a rotary motion driven pulley 50210 to rotate, to drive the rotation shaft 50206 to rotate. Because the rotation shaft 50206 is fixedly connected to the gripping jaw retractable motion assembly such as the retractable motion motor 50203 and the like, the gripping jaw 50201 can rotate. The retractable motion motor 50203 controls extension or retraction of the gripping jaw 50201, and a retractable motion motor gear 50211 is used to drive the gripping jaw retractable rack 50202 to make a retractable motion. Therefore, the gripping jaw 50201 can make a retractable motion, as shown in FIG 8.

The manipulator and transit module 5 is mainly used for conveying the reaction cup 9 to a required module position in this embodiment.

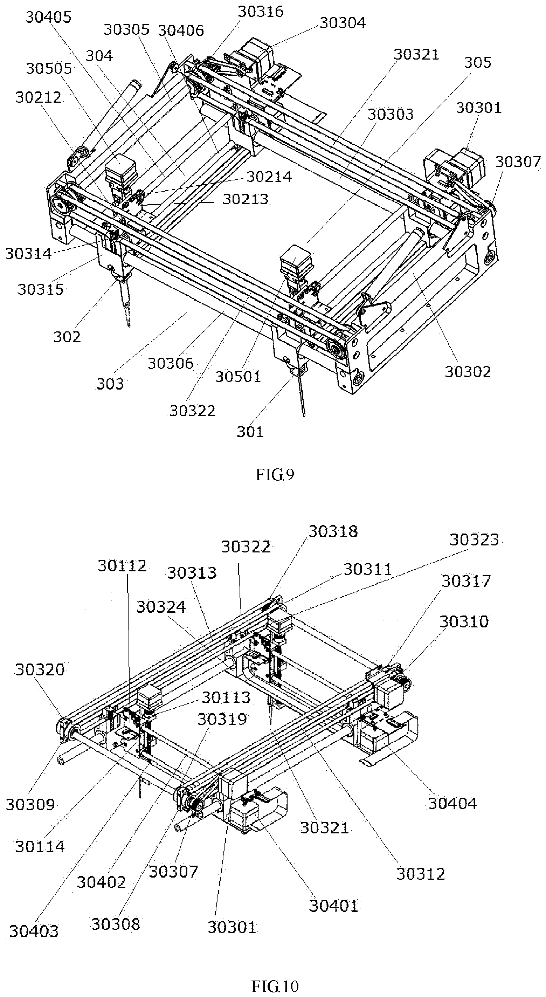

As shown in FIG 9, the robot-arm sample injection module 3 includes a first sampling device 301 disposed with a sampling needle assembly 30101 and a second sampling device 302 disposed with a pipette tip assembly 30201. The first sampling device 301 is used for drawing the reaction reagent and adding it to the reaction cup 9, and the second sampling device 302 is used for drawing the sample and adding it to the reaction cup 9. The robot-arm sample injection module 3 further includes a transverse motion assembly 303, a longitudinal motion assembly 304, and an up-and-down motion assembly 305, where the transverse motion assembly 303 drives the first sampling device 301 and the second sampling device 302 separately to make a transverse motion, the longitudinal motion assembly 304 drives the first sampling device 301 and the second sampling device 302 separately to make a longitudinal motion, and the up-and-down motion assembly 305 drives the first sampling device 301 and the second sampling device 302 separately to make an up-and-down motion.

The transverse motion assembly 303 includes a first transverse motion motor 30301 which drives a first transmission shaft 30302 to rotate, and drives the first sampling device 301 via the first transmission shaft 30302 to move transversely on a first transverse motion shaft 30303. The transverse motion assembly 303 further includes a second transverse motion motor 30304 which drives a second transmission shaft 30305 to rotate and drives the second sampling device 302 via the second transmission shaft 30305 to move transversely on a second transverse motion shaft 30306.

The first transverse motion motor 30301 and the second transverse motion motor 30304 in this embodiment are stepping motors. For the first sampling device 301, as shown in FIGs. 9 and 10, when the first transverse motion motor 30301 is started, a motor shaft drives a first synchronous pulley 30307 to rotate, thus driving the first transmission shaft 30302 to rotate; then, the first transmission shaft 30302 drives a second synchronous pulley 30308 and a fourth synchronous pulley 30309 to rotate; and meanwhile, the second synchronous pulley 30308 and the fourth synchronous pulley 30309 drive a seventh synchronous pulley 30310 and a ninth synchronous pulley 30311 to rotate. The seventh synchronous pulley 30310 and the ninth synchronous pulley 30311 are driven pulleys relative to the second synchronous pulley 30308 and the fourth synchronous pulley 30309. A connecting belt of the second synchronous pulley 30308 and the seventh synchronous pulley 30310 is a first synchronous belt 30312; and a connecting belt of the fourth synchronous pulley 30309 and the ninth synchronous pulley 30311 is a third synchronous belt 30313. The first sampling device 301 is driven via the first synchronous belt 30312 and the third synchronous belt 30313 to move transversely on the second transverse motion shaft 30306 and the first transverse motion shaft 30303.

The first synchronous belt 30312 and the third synchronous belt 30313 are separately connected to a spring clamp 30314. As shown in FIGs. 9 and 10, each spring clamp 30314 is connected to a spring clamp fixing block 30315. The transverse motion shafts (the second transverse motion shaft 30306 and the first transverse motion shaft 30303) penetrate through the spring clamp fixing block 30315, such that the spring clamp fixing block 30315 can slide on the transverse motion shafts. Further, the spring clamp fixing block 30315 is fixedly connected to two longitudinal motion shafts. That is, two ends of a first longitudinal motion shaft 30402 and a second longitudinal motion shaft 30403 are respectively connected to the two spring clamp fixing blocks 30315 on the first synchronous belt 30312 and the third synchronous belt 30313. A first longitudinal motion connection block 30114 is connected on the two longitudinal motion shafts, namely, the first longitudinal motion shaft 30402 and the second longitudinal motion shaft 30403 in a penetration manner, and can slide on the first longitudinal motion shaft 30402 and the second longitudinal motion shaft 30403; and the first sampling device 301 is connected thereon. A first PCB board 30112 is connected on the first longitudinal motion connection block 30114, and a pressure sensor 30113 is mounted on the first PCB board 30112.

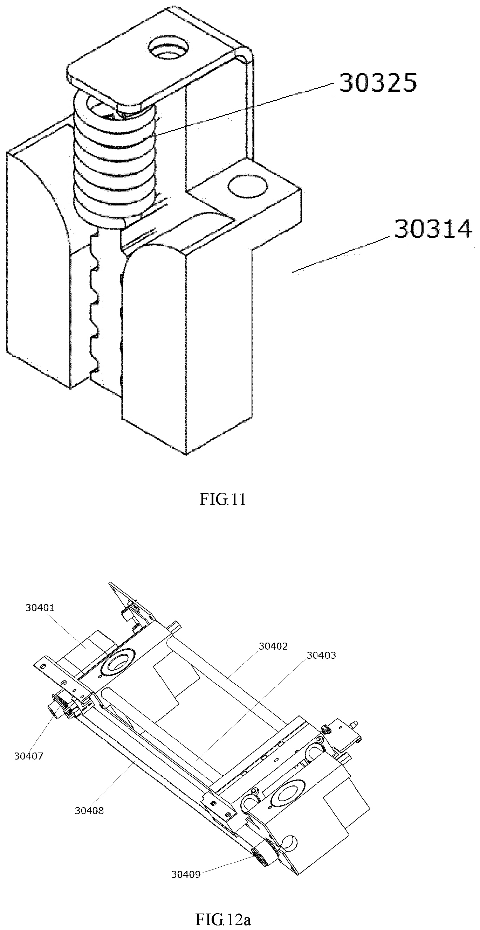

It should be noted that, the spring clamp 30314 is used for fixing the synchronous belts, and is connected onto a spring clamp fixing block 30324 (shown in FIG 10). The longitudinal motion shafts are connected on the spring clamp fixing block 30324, the first longitudinal motion connection block 30114 is connected on the longitudinal motion shafts, and the first sampling device 301 is connected on the first longitudinal motion connection block 30114. The spring clamp 30314 has a spring 30325, and a specific structure is shown in FIG 11. A connection manner between the spring clamp 30314 and the synchronous belts is an open connection, thus facilitating adjustment of the belts, maintaining belt tension and smooth running, and making it easy to replace the belts in the case of damage. A closed connection manner easily results in tension relaxation after long-time use, and leads to unsmooth long-term running.

Likewise, a second transverse motion motor 30304 controls a transverse motion of the second sampling device 302. Specifically, when the second transverse motion motor 30304 is started, a motor shaft drives a sixth synchronous pulley 30316 to rotate, thus driving a second transmission shaft 30305 to rotate; then, the second transmission shaft 30305 drives an eighth synchronous pulley 30317 and a tenth synchronous pulley 30318 to rotate; and meanwhile, the eighth synchronous pulley 30317 and the tenth synchronous pulley 30318 drive a third synchronous pulley 30319 and a fifth synchronous pulley 30320 to rotate. The third synchronous pulley 30319 and the fifth synchronous pulley 30320 are driven pulleys relative to the eighth synchronous pulley 30317 and the tenth synchronous pulley 30318. A connecting belt of the third synchronous pulley 30319 and the eighth synchronous pulley 30317 is a second synchronous belt 30321; and a connecting belt of the fifth synchronous pulley 30320 and the tenth synchronous pulley 30318 is a fourth synchronous belt 30322. The second sampling device 302 is driven via the second synchronous belt 30321 and the fourth synchronous belt 30322 to move transversely on the second transverse motion shaft 30306 and the first transverse motion shaft 30303.

The second synchronous belt 30321 and the fourth synchronous belt 30322 are separately connected to a spring clamp 30323 (having the same structure as the spring clamp 30314). As shown in FIGs. 9 and 10, each spring clamp 30323 is connected to a spring clamp fixing block 30324. The transverse motion shafts penetrate through the spring clamp fixing block 30324, such that the spring clamp fixing block 30324 can slide on the transverse motion shafts. Further, the spring clamp fixing block 30324 is fixedly connected to two longitudinal motion shafts. That is, two ends of a third longitudinal motion shaft 30405 and a fourth longitudinal motion shaft 30406 are respectively connected to the two spring clamp fixing blocks 30324 (or the spring clamp fixing blocks 30315) on the second synchronous belt 30321 and the fourth synchronous belt 30322. A second longitudinal motion connection block 30212 is connected on the two longitudinal motion shafts, namely, the third longitudinal motion shaft 30405 and the fourth longitudinal motion shaft 30406 in a penetration manner, and can slide on the third longitudinal motion shaft 30405 and the fourth longitudinal motion shaft 30406; and the second sampling device 302 is connected thereon. A third PCB board 30213 is connected on the second longitudinal motion connection block 30212, and a pressure sensor 30214 is mounted on the third PCB board 30213.

As shown in FIGs. 9 and 10, the longitudinal motion assembly 304 includes a first longitudinal motion motor 30401 which drives the first sampling device 301 to move longitudinally along a first longitudinal motion shaft 30402 and a second longitudinal motion shaft 30403. The longitudinal motion assembly further includes a second longitudinal motion motor 30404 which drives the second sampling device 302 to move longitudinally along a third longitudinal motion shaft 30405 and a fourth longitudinal motion shaft 30406.

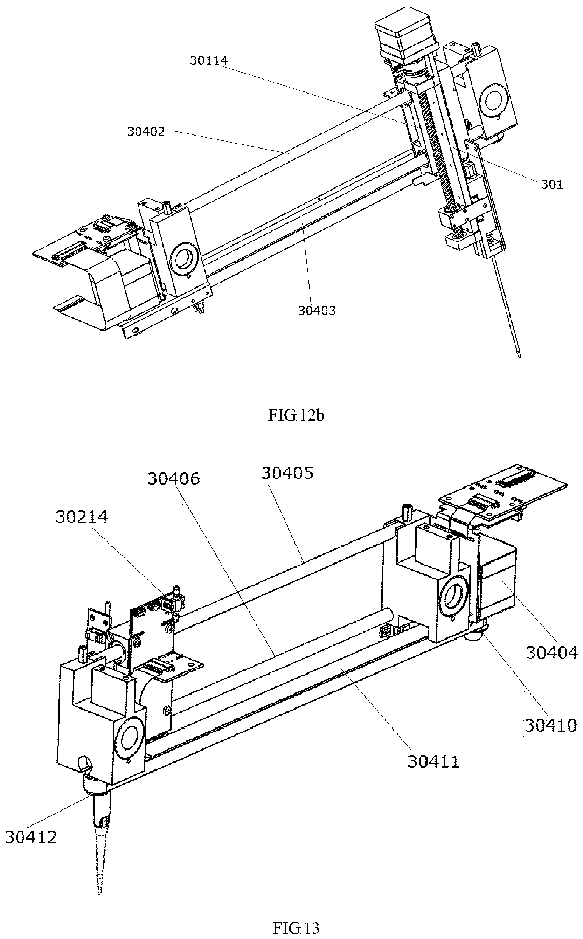

The first longitudinal motion motor 30401 controls a longitudinal motion of the first sampling device 301. Specifically, as shown in FIGs. 12a and 12b, when the first longitudinal motion motor 30401 is started, the motor drives a first driving pulley 30407 to rotate, and the first driving pulley 30407 drives a first driven pulley 30409 via a fifth belt 30408 to rotate, such that the first sampling device 301 can move along the first longitudinal motion shaft 30402 and the second longitudinal motion shaft 30403. The two ends of the first longitudinal motion shaft 30402 and the second longitudinal motion shaft 30403 are respectively connected to the spring clamp fixing blocks 30324 (or the spring clamp fixing blocks 30315), and the first sampling device 301 is connected on the first longitudinal motion shaft 30402 and the second longitudinal motion shaft 30403 via the longitudinal motion connection block 30114.

Likewise, the second longitudinal motion motor 30404 controls a longitudinal motion of the second sampling device 302. Specifically, as shown in FIG 13, when the second longitudinal motion motor 30404 is started, the motor drives a second driving pulley 30410 to rotate, and the second driving pulley 30410 drives a second driven pulley 30412 via a sixth belt 30411 to rotate, such that the second sampling device 302 can move along the third longitudinal motion shaft 30405 and the fourth longitudinal motion shaft 30406. The two ends of the third longitudinal motion shaft 30405 and the fourth longitudinal motion shaft 30406 are respectively connected to the spring clamp fixing blocks, and the second sampling device 302 is connected on the third longitudinal motion shaft 30405 and the fourth longitudinal motion shaft 30406 via the longitudinal motion connection block 30212.

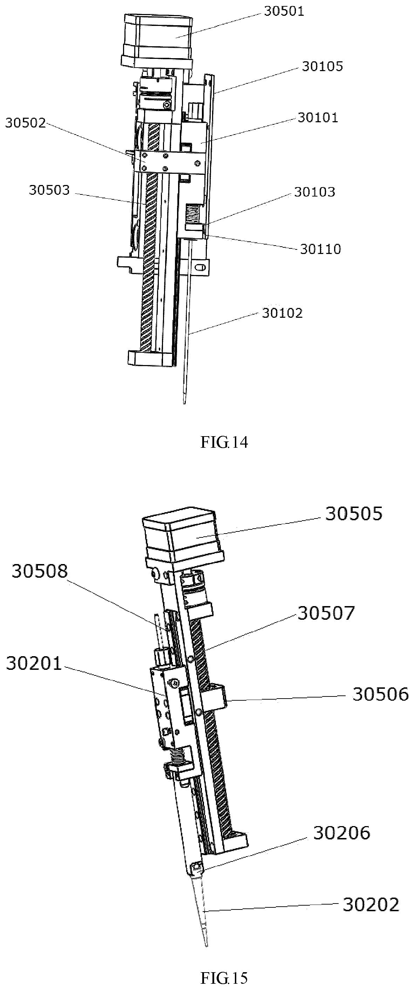

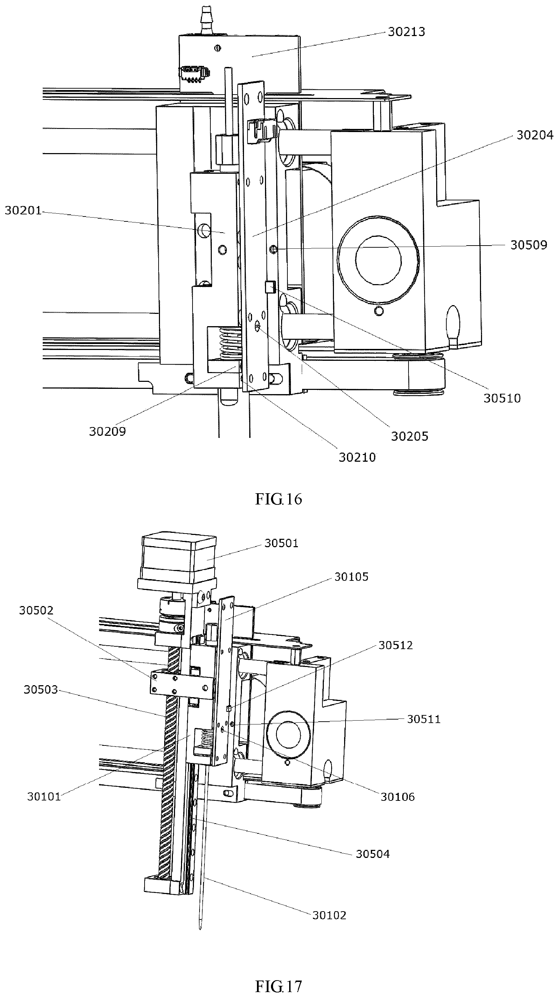

As shown in FIG 9, the up-and-down motion assembly 305 includes a first up-and-down motion motor 30501. As shown in FIG 14, the sampling needle assembly 30101 and a first up-and-down motion slider 30502 are connected, and the first up-and-down motion slider 30502 is sleeved on a first up-and-down motion screw rod 30503. The first up-and-down motion motor 30501 drives the first up-and-down motion screw rod 30503 to move, and the first up-and-down motion slider 30502 on the first up-and-down motion screw rod 30503 drives the sampling needle assembly 30101 to move up and down along a first guide rail 30504 (as shown in FIG 17). The up-and-down motion assembly further includes a second up-and-down motion motor 30505. As shown in FIG 15, the pipette tip assembly 30201 and a second up-and-down motion slider 30506 are connected, and the second up-and-down motion slider 30506 is sleeved on a second up-and-down motion screw rod 30507. The second up-and-down motion motor 30505 drives the second up-and-down motion screw rod 30507 to move, and the second up-and-down motion slider 30506 on the second up-and-down motion screw rod 30507 drives the pipette tip assembly 30201 to move up and down along a second guide rail 30508.

As shown in FIG 14, the first up-and-down motion motor 30501 controls an up-and-down motion of the first sampling device 301. When started, the first up-and-down motion motor 30501 drives the first up-and-down motion screw rod 30503 to move. Because the first up-and-down motion screw rod 30503 is slidably connected to the first up-and-down motion slider 30502 and the sampling needle assembly 30101 is fixedly connected to the first up-and-down motion slider 30502, the first up-and-down motion slider 30502 drives the sampling needle assembly 30101 to move up and down along the first guide rail 30504.

Likewise, as shown in FIG 15, the second up-and-down motion motor 30505 controls an up-and-down motion of the second sampling device 302. When started, the second up-and-down motion motor 30505 drives the second up-and-down motion screw rod 30507 to move. Because the second up-and-down motion screw rod 30507 is slidably connected to the second up-and-down motion slider 30506 and the pipette tip assembly 30201 is fixedly connected to the second up-and-down motion slider 30506, the second up-and-down motion slider 30506 drives the pipette tip assembly 30201 to move up and down along the second guide rail 30508.

As shown in FIG 16, during the up-and-down motion, positioning of the pipette tip assembly 30201 is realized by means of a second limit magnet 30509 and a second limit magnetic field sensing element 30510, where the second limit magnet 30509 is disposed on a side of the pipette tip assembly 30201 on the longitudinal motion connection block, and the second limit magnetic field sensing element 30510 is disposed on a side of the pipette tip assembly 30201 that is opposite the second limit magnet 30509. Likewise, during the up-and-down motion, positioning of the sampling needle assembly 30101 is realized by means of a first limit magnet 30511 and a first limit magnetic field sensing element 30512, where the first limit magnet 30511 is disposed on a side of the sampling needle assembly 30101 on the longitudinal motion connection block, and the first limit magnetic field sensing element 30512 is disposed on a side of the sampling needle assembly 30101 that is opposite the first limit magnet 30511, as shown in FIG 17.

The function of the robot-arm sample injection module 3 in this embodiment is as follows: The second sampling device 302 disposed with the pipette tip assembly 30201 draws up the sample via the pipette tip and adds it to the reaction cup; and the first sampling device 301 disposed with the sampling needle assembly 30101 draws up the reaction reagent and adds it to the reaction cup for reaction with the sample.

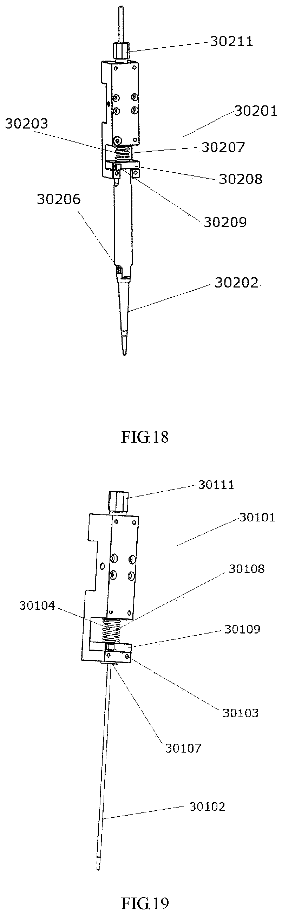

As shown in FIG 18, the pipette tip assembly 30201 has a pipette tip rod, of which a bottom end is used for picking up the pipette tip 30202. Further, the pipette tip rod is a second capacitance probe 30203, is connected to a fourth PCB board 30204 via a second elastic copper sheet 30205 (as shown in FIG 16), and is used for monitoring of the liquid level when the pipette tip 30202 starts to draw the liquid, where the foremost end of the pipette tip 30202 enters the sample liquid at a certain distance and then starts drawing the liquid. A pipette tip engaging portion 30206 is connected to a second spring 30207 and a second connection block 30208. When the pipette tip 30202 is picked up, the second spring 30207 is compressed and a pipette tip pickup confirmation magnet 30209 on the second connection block 30208 moves with the spring; and a magnetic field sensing element 30210 on the fourth PCB board 30204 senses changes in the magnetic field, to monitor and confirm whether the pipette tip 30202 is picked up.

A pipette tip liquid contact 30211 on the top of the pipette tip assembly 30201 is connected to a pressure sensor 30214 on the third PCB board 30213 (as shown in FIG 16), where the third PCB board 30213 is disposed on a second longitudinal motion connection block 30212 on the third longitudinal motion shaft 30405 and is used for monitoring a liquid pressure in the pipette tip 30202. The pipette tip 30202 moves to the upper side of a reaction cup carrier after drawing the liquid, and discharges the liquid to the reaction cup 9. After its interior is emptied, the second spring 30207 is reset, such that the waste pipette tip 30202 is discarded to a waste bin 309 (as shown in FIG 1).

The function of a needle point protection magnet 30103 of the sampling needle assembly 30101 is as follows: When a sampling needle 30102 touches the bottom of a sample tube, the first sampling device 301 is stopped from continuously moving downwards by means of magnetic induction, thus protecting the point of the sampling needle 30102.

The sampling needle assembly 30101 has the following specific structure: As shown in FIG 19, the sampling needle assembly 30101 has a sampling needle rod disposed with the sampling needle 30102 on the bottom end. To realize an integrated design, the sampling needle rod itself is also a first capacitance probe 30104, is connected to a second PCB board 30105 via a first elastic copper sheet 30106 (as shown in FIG 17), and is used for monitoring of the liquid level when the sampling needle 30102 starts to draw the liquid, where the foremost end of the sampling needle 30102 enters the reaction reagent at a certain distance and then starts drawing the liquid. A sampling needle fitting joint 30107 is disposed on the sampling needle rod, and is connected to a first spring 30108 and a first connection block 30109. When the sampling needle 30102 touches the kit bottom during liquid drawing, the first spring 30108 is compressed and a needle point protection magnet 30103 on the first connection block 30109 moves with the spring; and a magnetic field sensing element 30110 on the second PCB board 30105 senses changes in the magnetic field. Then the sampling needle 30102 stops moving downwards, to protect the needle point from damage, as shown in FIG 14.

A sampling needle liquid contact 30111 on the top of the sampling needle assembly 30101 is connected to a pressure sensor 30113 on the first PCB board 30112 (as shown in FIG 10), where the first PCB board 30112 is disposed on the first longitudinal motion connection block 30114 on the first longitudinal motion shaft 30402 and is used for monitoring a liquid pressure in the sampling needle 30102. The sampling needle 30102 moves to the upper side of the reaction cup carrier after drawing the liquid, and discharges the internal liquid to the reaction cup 9.

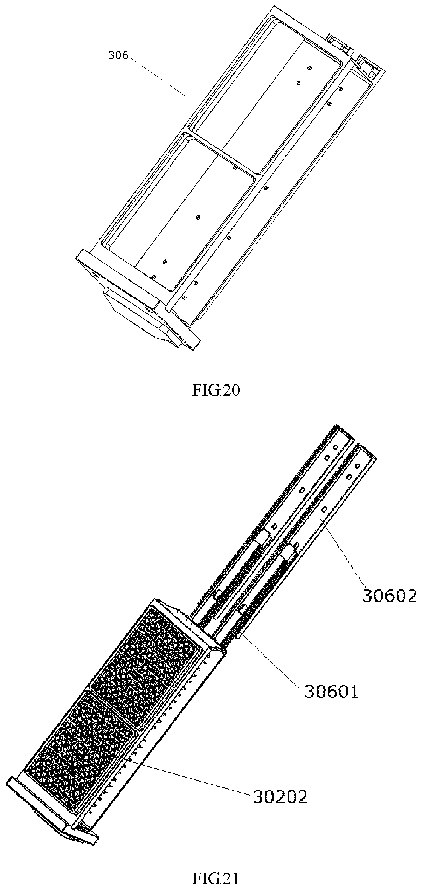

In addition, as shown in FIG 20, to cooperate with the second sampling device 302, the robot-arm sample injection module 3 of this embodiment further includes a pipette tip holder 306, where the bottom of the pipette tip holder 306 is disposed with a pipette tip cassette rail 30601. As shown in FIG 21, the pipette tip holder 306 slides on the pipette tip cassette rail 30601 via a pipette tip cassette in a fitted manner, and then slides on a pipette tip holder rail 3062 in a fitted manner. Through the two slide rails, the pipette tip holder 306 can extend out of the instrument to replace the pipette tip 30202.

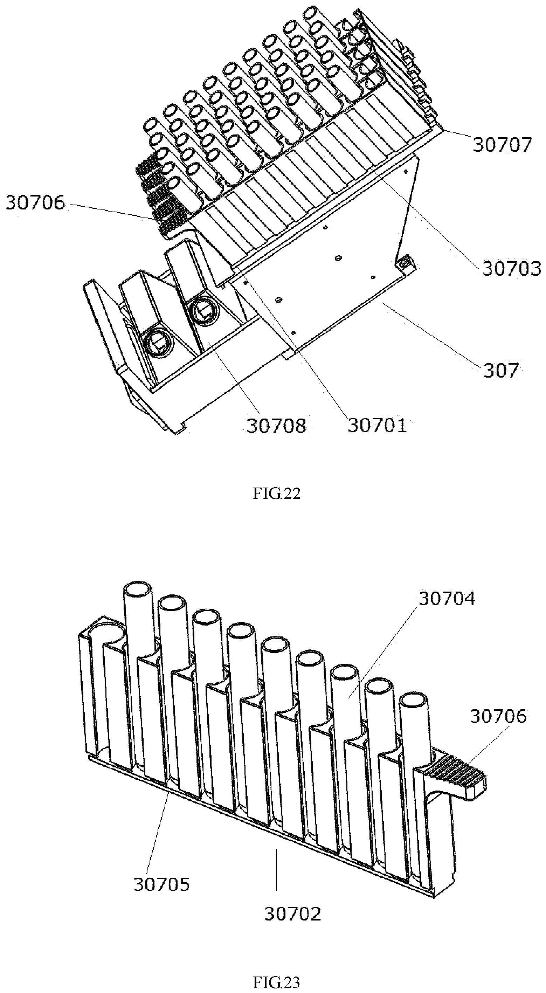

In this embodiment, the robot-arm sample injection module 3 is equipped with a sample tube bin assembly 307. As shown in FIG 22, the sample tube bin assembly 307 is provided with a sample tube rest 30701 (as shown in FIG 23), a plurality of sample tube cassettes 30702 is disposed on the sample tube rest 30701, and a plurality of sample tubes 30704 can be placed in the sample tube cassettes 30702. An elastic steel ball part 30703 (at the shown position) is disposed on the surface of the sample tube rest 30701, and a steel ball fitting groove 30705 (at the shown position) that fits the elastic steel ball part 30703 is provided on a corresponding position on the bottom of each sample tube cassette 30702. Projections 30707 are disposed on the bottom of the front end (at a position far away from a handle) of the sample tube rest 30701; and a front portion of the sample tube bin assembly 307 is further disposed with an trigger solution storage box 30708. During use, by means of the elastic steel ball part 30703 disposed on the surface of the sample tube rest 30701 and the steel ball fitting groove 30705 on a corresponding position on the bottom of each sample tube cassette 30702, the steel ball bounces up for securing when the sample tube cassette is pushed in. By means of the projections 30707 on the bottom of the front end (at a position far away from the handle 30706) of the sample tube rest 30701, the front end senses the resistance when the sample tube cassette 30702 is pushed in, which indicates that the sample tube cassette 30702 is mounted in place.

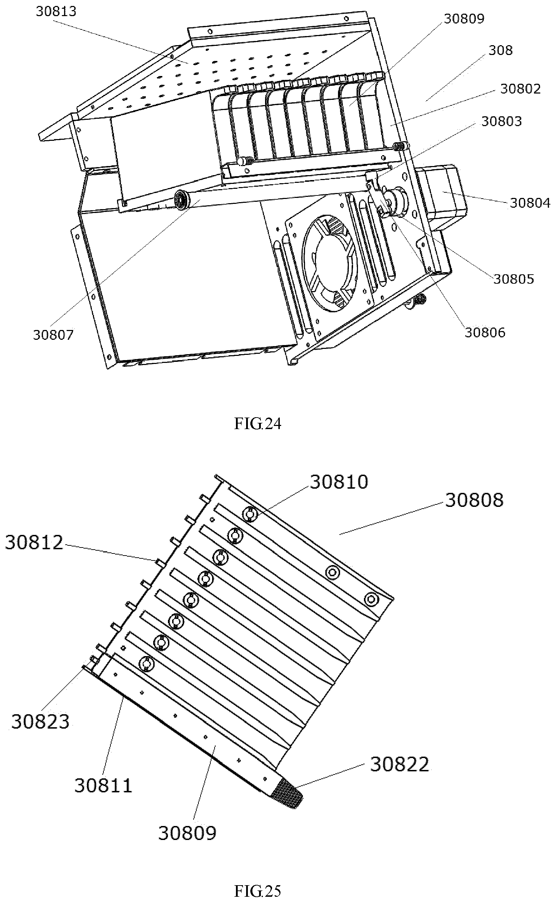

In this embodiment, the robot-arm sample injection module 3 is further equipped with a kit bin 308. As shown in FIG 24, the kit bin 308 includes a cover plate motion assembly 30801, a kit assembly 30802, a kit holder connecting block 30803, a kit mixing motor 30804, a motor rotation connecting block 30805, a connecting rod 30806, and a mixing support shaft 30807. The kit assembly 30802 includes a kit holder 30808 which is provided with several kits 30809. A contact face between the kit holder 30808 and each kit 30809 is disposed with an elastic steel ball part 30810, and a steel ball fitting groove 30811 (at the shown position) is provided on the bottom of each corresponding kit 30809. When each kit 30809 is pushed into the front end of the kit holder 30808, the steel ball bounces up to secure the kit 30809. Further, as shown in FIG 25, projections 30812 are disposed on the edge of the front end of the kit holder 30808. When the kit 30809 is pushed in, the front end senses the resistance, which indicates that the kit 30809 is mounted in place. The kit 30809 has a handle 30822 and projections 30823.

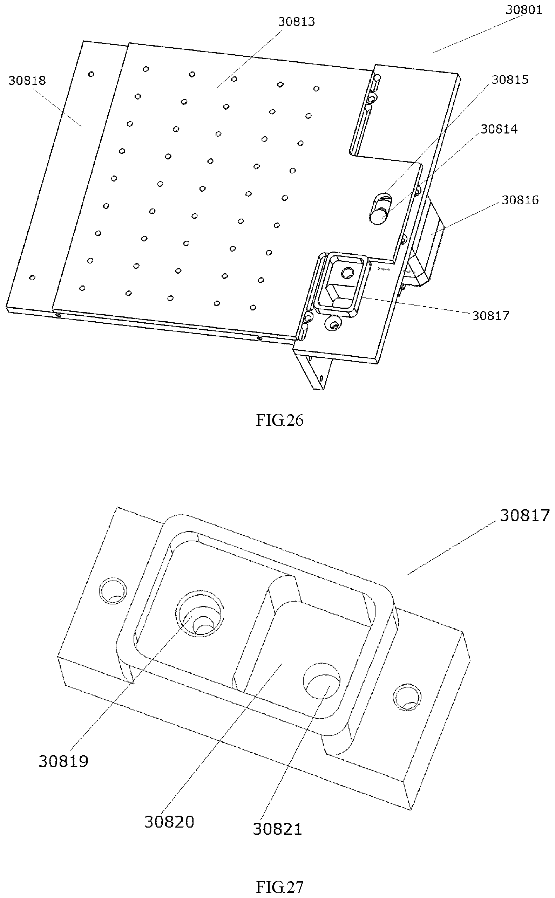

As shown in FIG 26, the cover plate motion assembly 30801 includes a cover plate 30813, a motor rotation connecting block 30814, a cover plate movement fitting groove 30815, a cover plate motion motor 30816, a sampling needle cleaning pool 30817, and a sampling isolation plate 30818. The motion principle of the cover plate motion assembly 30801 is as follows: When the cover plate motion motor 30816 is started, the motor rotation connecting block 30814 rotates to drive the cover plate 30813 to move back and forth with the synergy of the cover plate movement fitting groove 30815. When it is not required to transfer the liquid, the cover plate 30813 moves backwards to cover the sample and the reaction reagent, such that the reagent and the sample are prevented from pollution and the detection result is not affected.

The sample tube bin assembly 307 and the kit bin 308 are both disposed with a sampling isolation plate and a cover plate on the upper portion, where the cover plate is located above the sampling isolation plate, and sampling holes each with a size corresponding to the sample tubes and kit sampling ports are evenly distributed on both of the plates. When it is required to transfer the liquid, the sampling holes on the sampling isolation plate and those on the cover plate coincide up and down, and the sampling needle and the pipette tip may be inserted into the reaction reagent and the sample to transfer the liquid. After operation completion, the cover plate moves backwards with respect to the sampling isolation plate, and their sampling holes are misaligned, such that the sampling needle and the pipette tip cannot transfer the liquid.

As shown in FIG 27, the sampling needle cleaning pool 30817 includes a cleaning pool 30819 and a cleaning pool spare chamber 30820, and is used for cleaning the sampling needle 30102. The sampling needle cleaning pool 30817 consists of two parts: the cleaning pool 30819 and the cleaning pool spare chamber 30820, and both are provided with liquid discharge holes 30821 on the bottom. A cleaning solution enters the sampling needle 30102 from a liquid contact on the top of the sampling needle 30102 to clean the needle. The cleaning pool spare chamber 30820 is arranged such that the cleaning solution overflowing the cleaning pool 30819 can flow out from the liquid discharge holes 30821 on the bottom of the cleaning pool spare chamber 30820.

The kit 30809 has a uniform mixing function. The kit mixing motor 30804 rotates and drives the motor rotation connecting block 30805 thereon to rotate. The connecting rod 30806 is connected to the motor rotation connecting block 30805, and is further connected to the kit holder connecting block 30803 in a movable manner. The kit holder connecting block 30803 is fixed on the bottom surface of the kit holder 30808, and the bottom surface of the kit holder 30808 is fixedly connected to the mixing support shaft 30807. Therefore, the kit 30809 and the mixing support shaft 30807 swing together, to uniformly mix the reaction reagent in the kit 30809.

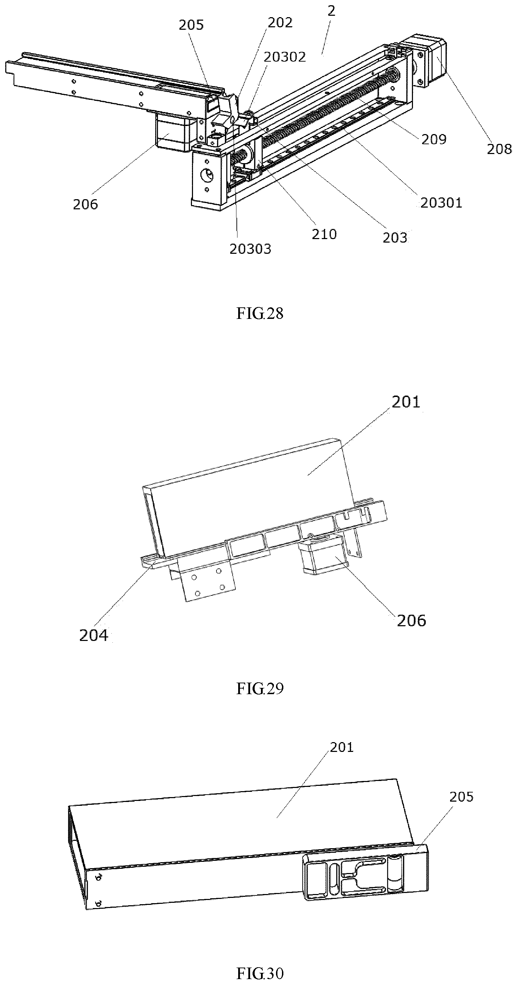

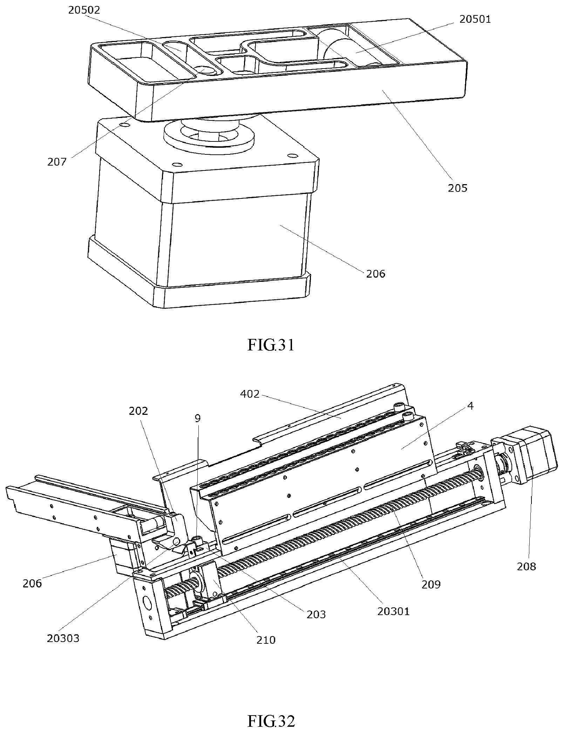

As shown in FIGs. 28 and 32, the reaction cup conveyance module 2 includes a reaction cup conveyance chamber 201 (as shown in FIG. 29), and the reaction cup 9 falling from the reaction cup conveyance chamber 201 is adjusted in posture by means of a reaction cup flipper 202. The reaction cup conveyance module further includes a reaction cup carrier 203 which pushes the reaction cup 9 after posture adjustment to a designated position. A moving track of the reaction cup carrier 203 is below the incubation module 4.

The reaction cup conveyance chamber 201 slides in along a reaction cup cassette holder 204, and a reaction cup transfer bracket 205 (as shown in FIG. 30) is located on the bottom portion of the reaction cup conveyance chamber 201. A reaction cup transfer groove 20501 that fits the reaction cup cassette holder 204 is disposed on the reaction cup transfer bracket 205, and the reaction cup transfer bracket 205 moves back and forth with the synergy of a cup transfer fitting groove 20502 (as shown in FIG. 31). The reaction cup 9 in the reaction cup conveyance chamber 201 enters the reaction cup transfer groove 20501 and then falls into the reaction cup flipper 202.

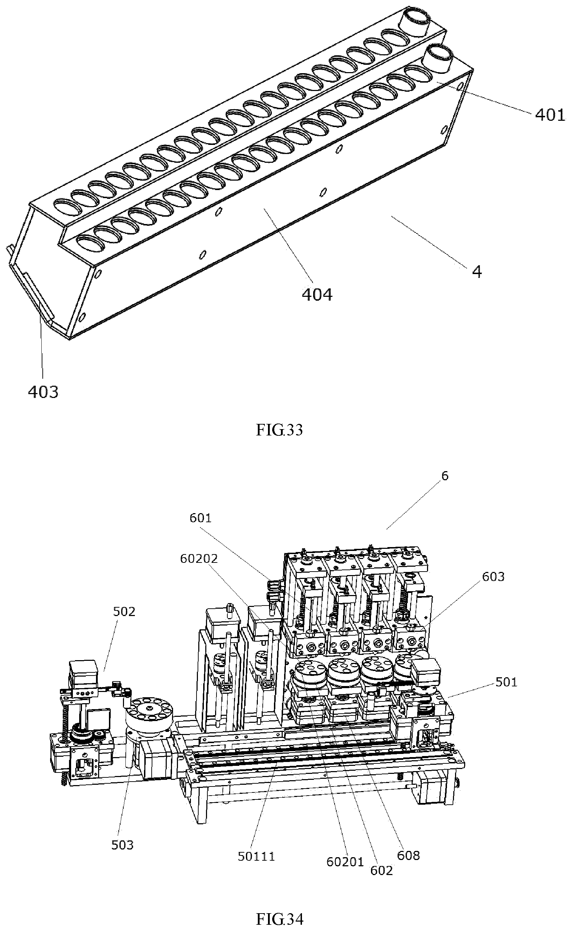

The robot-arm sample injection module 3 adds the reaction reagent and the sample separately to the reaction cup 9 which is pushed by the reaction cup carrier 203 to the designated position, and then the manipulator and transit module 5 carries the reaction cup 9 into an incubation base 401 of the incubation module 4. The incubation base 401 is secured on an incubation base vertical panel 402 via nuts, where an incubation sheet 403 is disposed on the bottom of the incubation base 401 and insulating sheets 404 are respectively disposed at the two sides of the incubation base 401, as shown in FIG. 33.

In this embodiment, the reaction cup 9 lies in (in a horizontal position) the reaction cup conveyance chamber 201 and slides in along the reaction cup cassette holder 204. The reaction cup transfer bracket 205 is located on the bottom portion of the reaction cup conveyance chamber 201, and its reaction cup transfer groove 20501 has a hollow-carved design at a position corresponding to the reaction cup cassette holder 204. When moving to this position, the reaction cup 9 goes into the reaction cup transfer groove 20501. A reaction cup transfer motor 206 rotates. As shown in FIG 31, located below the reaction cup transfer bracket 205, the reaction cup transfer motor 206 controls the motor rotation connecting block 207 to rotate, and drives the reaction cup transfer bracket 205 to move back and forth with the synergy of the cup transfer fitting groove 20502. When the reaction cup transfer bracket moves forwards, the reaction cup 9 falls to a place below the reaction cup transfer bracket 205 that is hollow-carved, and therefore, the reaction cup 9 falls into the reaction cup flipper 202 from this place. The reaction cup flipper 202 is controlled by a reaction cup conveyance motor 208 and a screw rod 209 rotates, and the reaction cup flipper 202 moves along the guide rail 20301 by means of a reaction cup carrier slider 210. By a reaction cup pusher 20302 on the front end of the reaction cup flipper 202, the reaction cup flipper 202 changes the reaction cup 9 from the previous lying posture to a standing posture by means of a flipper pivot 20303, such that the reaction cup 9 goes into the reaction cup carrier 203. A moving track of the reaction cup carrier 203 is below the incubation module 4, as shown in FIG 32.

When the reaction cup carrier 203 moves to the designated position, the sampling needle 30102 adds the reaction reagent to the reaction cup 9 and the pipette tip 30202 adds the sample to the reaction cup 9. Then the reaction cup carrier 203 moves to the designated position, and the first manipulator 501 carries the reaction cup 9 into the incubation base 401 of the incubation module 4, for an incubation reaction.

The reaction cup conveyance module 2 is mainly used to add the sample and the reaction reagent to the reaction cup 9 for an incubation reaction.

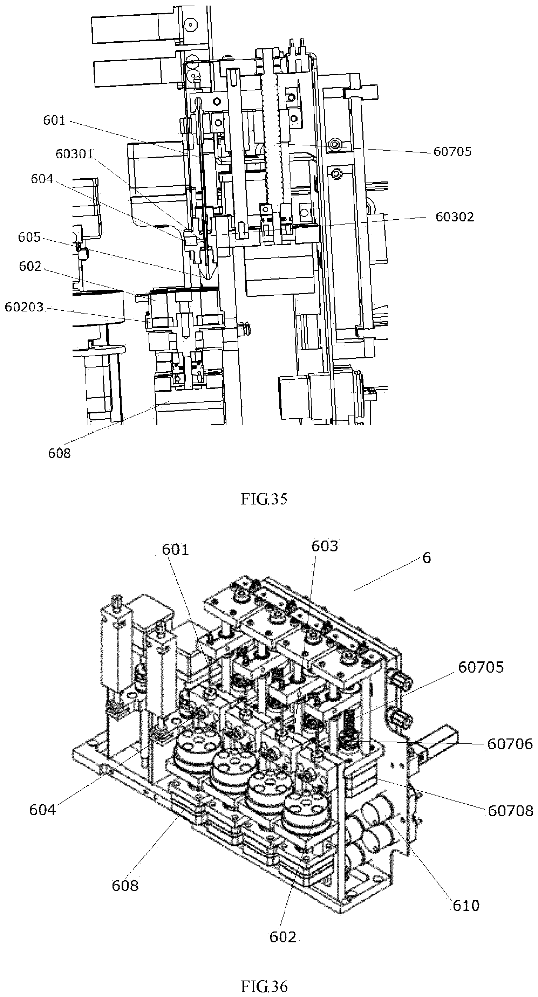

As shown in FIG 34, the magnetic-separation cleaning module 6 includes a magnetic-separation cleaning needle 601 (four magnetic-separation cleaning needles are provided in this embodiment), and a rotatable magnetic-separation seat 602 is disposed below the magnetic-separation cleaning needle 601, where the magnetic-separation seat 602 has at least one reaction cup hole 60201. In this embodiment, each magnetic-separation seat 602 is provided with four reaction cup holes 60201 and one spare reaction cup hole 60202. The magnetic-separation cleaning module further includes a magnetic cleaning pool 603. The magnetic cleaning pool 603 includes a cleaning solution path 60301 and a cleaning needle liquid discharge path 60302, where a cleaning solution enters through a cleaning solution inlet 604 in the cleaning solution path 60301 and then goes into the reaction cup 9 through a cleaning solution pointed outlet 605, to clean the reaction cup, as shown in FIG 35. The magnetic-separation cleaning needle 601 is used for discharging (from the position shown in FIG 35) the waste liquid in the reaction cup 9 after cleaning through the cleaning needle liquid discharge path 60302. As shown in FIG 36, the instrument is further disposed with a diaphragm pump 610 located below a magnetic-cleaning lifting motor 60708 and at the inner side of the magnetic-separation seat 602.

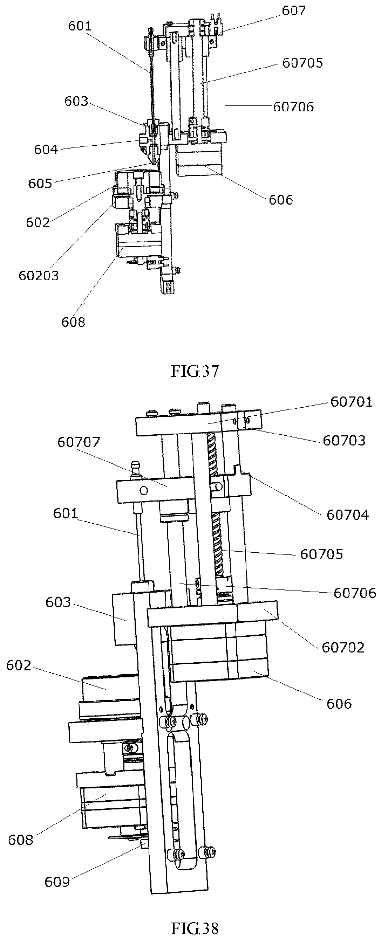

After the cleaning solution enters through the cleaning solution inlet 604, the magnetic-separation cleaning needle 601 is lifted up from the cleaning solution pointed outlet 605, and the cleaning solution enters the reaction cup 9 through the cleaning solution pointed outlet 605. While flowing through the cleaning solution pointed outlet 605, the cleaning solution can also clean the magnetic-separation cleaning needle 601. The magnetic-separation cleaning needle 601 is used for drawing and discharging the waste liquid in the reaction cup 9 after cleaning. The cleaning process includes 3 to 5 liquid introduction and discharge operations. The magnetic-separation seat 602 rotates and cleans the reaction cups successively, as shown in FIG 37. Components, such as the magnetic-separation cleaning needle 601, the magnetic-separation seat 602, the magnetic cleaning pool 603, and the magnetic-cleaning lifting motor 606, constitute a magnetic-separation cleaning device in the magnetic-separation cleaning module 6; and multiple magnetic-separation cleaning devices may be superposed and assembled according to the requirements of the instrument, as shown in FIGs. 34 and 36.

An up-and-down motion of the magnetic-separation cleaning needle 601 is controlled by the magnetic-cleaning lifting motor 606. As shown in FIG. 38, the magnetic-separation cleaning module 6 is further disposed with a lifting limit structure 607. The lifting limit structure 607 includes a magnetic-cleaning lifting upper plate 60701 and a magnetic-cleaning lifting lower plate 60702; and a limit optocoupler 60703, a cleaning needle lifting stopper 60704, a lifting screw rod 60705, and a guide rod 60706 are disposed between the magnetic-cleaning lifting upper plate 60701 and the magnetic-cleaning lifting lower plate 60702. A slider 60707 is slidably connected on the lifting screw rod 60705, and the magnetic-cleaning lifting motor 60708 drives the lifting screw rod 60705 to rotate. Because the slider 60707 is connected to the guide rod 60706 and the magnetic-separation cleaning needle 601, the magnetic-separation cleaning needle 601 is driven by the slider 60707 to move up and down along the guide rod 60706.

The magnetic-separation seat 602 is driven by a magnetic-separation seat rotary motor 608 to rotate, and a rotation limit structure 609 is disposed below the magnetic-separation seat rotary motor 608. A magnet 60203 is disposed inside the magnetic-separation seat 602 (as shown in FIG. 35).

A rotary motion of each magnetic-separation seat 602 is controlled by the magnetic-separation seat rotary motor 608, and each magnetic-separation seat 602 is provided with four reaction cup holes 60201 and one spare reaction cup hole 60202. The number of the magnetic-separation seats 602 is set as required. The magnet 60203 is disposed in the lower portion of each reaction cup seat, and is used for adsorbing a target substance to be tested in the sample.

The magnetic-separation cleaning module 6 is mainly used for magnetic-separation cleaning of a detection target, such that the cleaned target waits for entering the detection module 7 for detection.

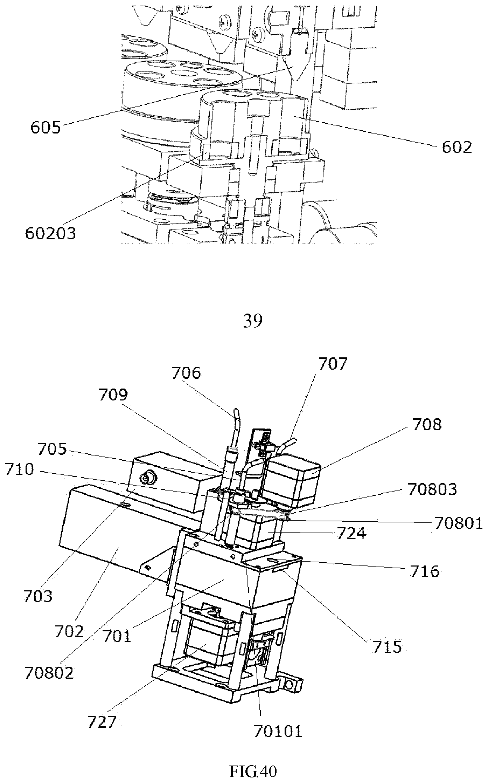

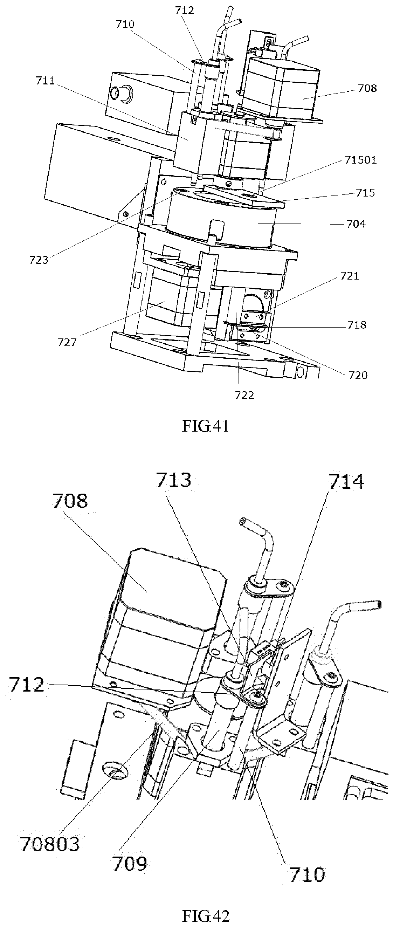

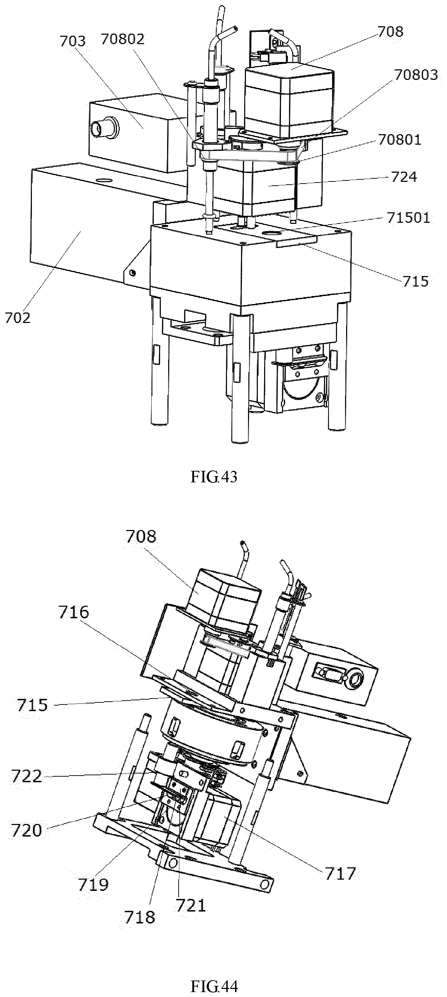

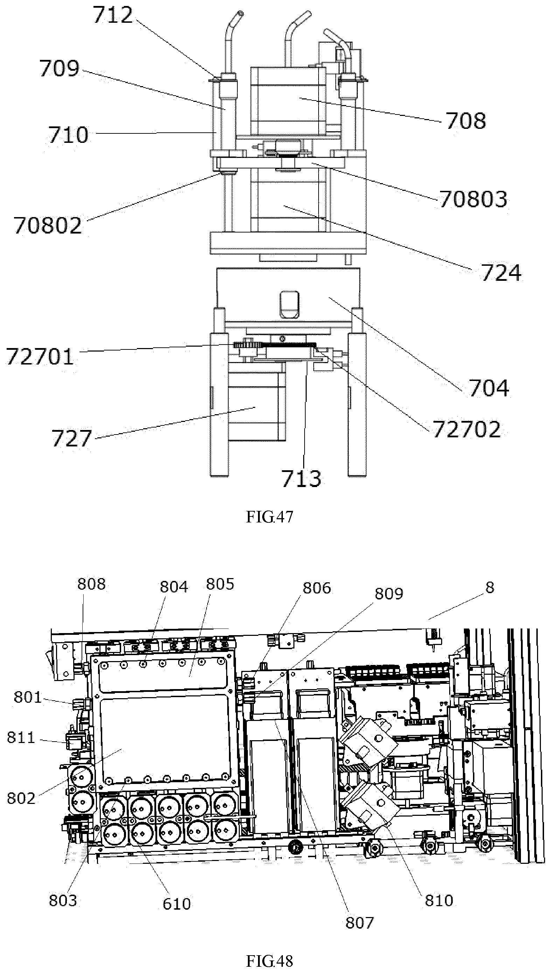

As shown in FIGs. 40 and 43, the detection module 7 includes a detection chamber 701, a PMT 702, and a photon counter 703. The reaction cup 9 is carried by the manipulator and transit module 5 to a reaction cup detection seat 704 of the detection chamber 701 (as shown in FIG. 41). A first trigger solution and a second trigger solution are successively added to the reaction cup 9 respectively through a first trigger solution pipeline 705 and a second trigger solution pipeline 706, and after reaction, the waste liquid is discharged through a waste liquid discharge pipeline 707 of the detection module 7. Then, the PMT 702 and the photon counter 703 are used to collect and convert optical signals into electrical signals, to finally obtain a detection result through analysis. In this embodiment, specifically, the reaction cup 9 is carried by the second manipulator 502 in the manipulator and transit module 5 from the transit device 503 into the reaction cup detection seat 704 by means of a detection chamber baffle 715 and a reaction cup lift rod 722 (as shown in FIG 41). A reaction cup detection seat rotary motor 727 rotates, which, as shown in FIG. 47, is disposed with a rotation driving pulley 72701 with a gear; and the rotation driving pulley 72701 drives a rotation driven pulley 72702 located below the reaction cup detection seat. Thus, the reaction cup 9 is driven to rotate; and the first trigger solution pipeline 705, the second trigger solution pipeline 706, and the waste liquid discharge pipeline 707 move up and down, to add the trigger solutions 1 and 2 successively to the reaction cup 9 and discharge the waste liquid after reaction.

The detection module 7 further includes a motor 708 for trigger solution refilling and waste liquid tube lifting motions, which can drive simple screw rods 709 to move up and down. The simple screw rods 709 are slidably connected to detection module guide rods 710, to drive the first trigger solution pipeline 705, the second trigger solution pipeline 706, and the waste liquid discharge pipeline 707 to respectively move up and down along the detection module guide rods 710 (it should be noted that, the first trigger solution pipeline 705, the second trigger solution pipeline 706, and the waste liquid discharge pipeline 707 are each equipped with a simple screw rod and a detection module guide rod). The detection module guide rods 710 are connected to a guide rod fixing block 711, and are further respectively connected to the first trigger solution pipeline 705, the second trigger solution pipeline 706, and the waste liquid discharge pipeline 707 through a connecting piece 712. A limit stopper 713 is disposed on the connecting piece 712 and cooperates with an optocoupler 714, for limiting an upward motion, as shown in FIG 42. Specifically, when the motor 708 for trigger solution refilling and waste liquid tube lifting motions is started, the driving pulley 70801 drives three driven pulleys 70802 (the three pipelines, namely, the first trigger solution pipeline 705, the second trigger solution pipeline 706, and the waste liquid discharge pipeline 707, are each equipped with a driven pulley) via a belt 70803 to rotate, such that the simple screw rods 709 move up and down. The simple screw rods 709 are slidably connected to the detection module guide rods 710, thus driving the three liquid pipelines to move up and down respectively along the detection module guide rods 710.

When the reaction cup 9 is carried by the manipulator and transit module 5 into the reaction cup detection seat 704 of the detection chamber 701, the detection chamber baffle 715 moves so that a reaction cup transfer hole 716 and a baffle through hole 71501 on the detection chamber baffle 715 coincide, and then the reaction cup 9 is put in. Further, because the reaction cup detection seat 704 is located deeply, the reaction cup lift rod 722 is required to lift up to take the reaction cup 9 downwards to a reaction cup detection position 723; and afterwards, the trigger solution is added. A motion principle of the reaction cup lift rod is as follows: As shown in FIGs. 44 and 41, when started, a reaction cup lifting control motor 717 drives a motor rotation connecting pillar 718 to rotate, to drive a lift rod carrier connecting block 719 to move up and down with the synergy of a lift rod movement fitting groove 720, where a lift rod carrier 721 is fixedly connected on the lift rod carrier connecting block 719. The lift rod carrier 721 is connected to the reaction cup lift rod 722, and the reaction cup lift rod 722 moves up and down to take the reaction cup 9 into the reaction cup detection position 723. It should be noted that, the diameter of a bottom hole of the reaction cup detection position 723 is smaller than that of the section of the reaction cup, and fits the diameter of the reaction cup lift rod 722. Therefore, the reaction cup lift rod 722 moves down and passes through the hole of the reaction cup detection position 723, to put the reaction cup 9 in place.

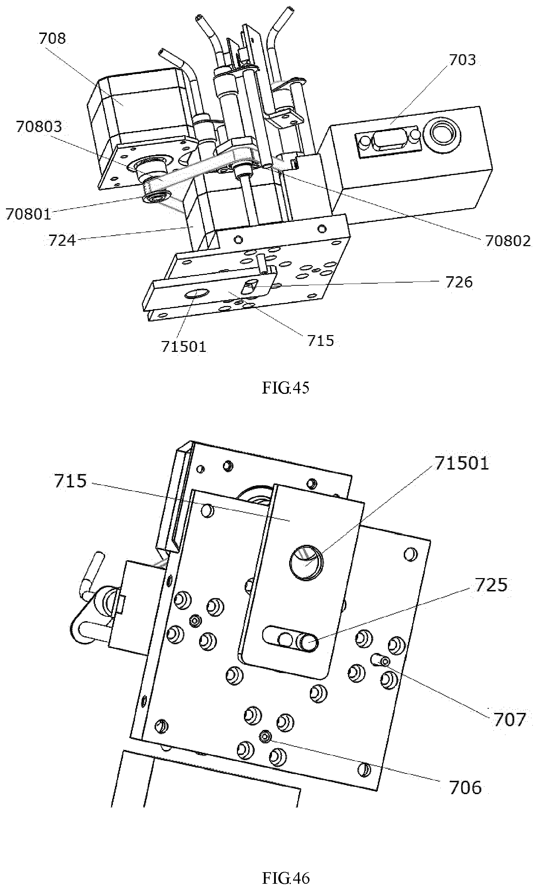

The detection chamber baffle 715 is mounted below a detection chamber top cover 70101, and a baffle motion motor 724 is started. As shown in FIG. 45 and 46, the baffle motion motor 724 drives a baffle motor rotation connecting pillar 725 to rotate, such that the detection chamber baffle 715 moves back and forth with the synergy of a baffle motion fitting groove 726, and accordingly, the baffle through hole 71501 on the detection chamber baffle 715 and the reaction cup transfer hole 716 coincide or are misaligned, to open or close the detection chamber 701, such that the detection chamber 701 is a dark room, thus guaranteeing a light avoidance reaction.

The first trigger solution pipeline 705, the second trigger solution pipeline 706, and the waste liquid discharge pipeline 707 all run through the detection chamber top cover 70101, as shown in FIG. 46. The trigger solution is added to the reaction cup 9 in the reaction cup detection seat 704. The waste liquid discharge pipeline 707 is relatively long, and can stick into the liquid level in the reaction cup for liquid drawing and discharge.

In this embodiment, the detection module 7 is mainly used to add the trigger solution to and discharge the waste liquid from the reaction cup 9 after magnetic cleaning, and then to conduct detection.

The fully-automatic chemiluminescence immunoassay analyzer of this embodiment further includes a circuit control module and a liquid path module 8, where the circuit control module is not described in detail herein, and persons skilled in the art can self-design the circuit control module according to the technical solution disclosed in the present invention without creative effort.

The liquid path module 8 of this embodiment includes some of the above-described modules or some components in the modules. Specifically, as shown in FIG 1, the liquid path module is integrally arranged on the rear side of the instrument and behind the magnetic-separation cleaning module 6. As shown in FIG 48, the liquid path module mainly includes: a magnetic-separation cleaning solution inlet path, a magnetic-separation cleaning needle discharge path, a sample and reaction reagent transfer path, a sampling needle cleaning path, an trigger solution inlet path, and a detection chamber liquid discharge path.

In the magnetic-separation cleaning solution inlet path, a cleaning solution is added from an external cleaning solution tank into a cleaning solution pool 802 through a cleaning solution pool entrance 801; flows to a diaphragm pump 610 through a cleaning solution pool branch outlet 803, and further to the cleaning solution inlet 604 of the magnetic cleaning pool 603 through a liquid path hose; and finally enters the reaction cup 9.

In the magnetic-separation cleaning needle discharge path, the waste liquid after cleaning is drawn by the magnetic-separation cleaning needle 601, flows through the diaphragm pump 610 via the liquid path hose, and enters a waste reservoir 805 through a waste reservoir branch inlet 804; and is finally discharged out of the instrument from a waste reservoir exit 806 to an external waste liquid tank.

In the sample and reaction reagent transfer path, the sample and the reaction reagent are drawn/discharged through a connected piston pump 807 by means of the pipette tip 30202 and the sampling needle 30102 of the robot-arm sample injection module 3.

In the sampling needle cleaning path, liquid transfer and cleaning paths for the sampling needle are switched via a solenoid valve 811. To clean the sampling needle of the robot-arm sample injection module 3, the cleaning solution is introduced from the cleaning solution pool 802 via a path same as the magnetic cleaning solution inlet path, flows through the diaphragm pump 610 and the piston pump 807 successively, and finally enters the sampling needle 30102. The waste liquid is discharged from the sampling needle cleaning pool 30817 through the diaphragm pump 610 to the waste reservoir 805, and is discharged out of the instrument via the waste reservoir exit 806 to the external waste liquid tank via a path same as the magnetic cleaning needle liquid path.

In the trigger solution inlet path, an trigger solution flows from the trigger solution storage box 30708 in an trigger solution bin, through an trigger solution dosing pump 810, and to the reaction cup 9 in the detection chamber 701 via an trigger solution pipeline, for reaction.

In the detection chamber liquid discharge path, after reaction, the liquid enters the waste reservoir 805 through the diaphragm pump 610 via a liquid discharge pipeline, and is discharged out of the instrument from the waste reservoir exit 806 to the external waste liquid tank.

In addition, some waste liquid may enter the waste reservoir 805 from the waste reservoir entrance 808. The liquid path module 8 is further provided with a cleaning solution pool exit 809 for discharge of the cleaning solution.

The basic principles, main features and advantages of the present invention have been shown and described above. Those skilled in the art should understand that the present invention is not limited by the foregoing embodiments, and the foregoing description in the embodiments and the specification are merely for explaining the principle of the present invention. Various changes and improvements may be made to the present invention without departing from the scope of the present invention. For example, various parts mounted on the base may be adjusted in position. These changes and improvements all fall within the protection scope of the present invention. The scope of protection claimed by the present invention is defined by the appended claims.

## Claims

1. A fully-automatic chemiluminescence immunoassay analyzer, comprising a base (1), wherein a reaction cup conveyance module (2), a robot-arm sample injection module (3), an incubation module (4), a manipulator and transit module (5), a magnetic-separation cleaning module (6), and a detection module (7) are mutually independently integrated on the base (1), wherein
the reaction cup conveyance module (2) is used for conveying a reaction cup (9) to a pre-determined position, such that the robot-arm sample injection module (3) is able to add a reaction reagent and a sample separately into the reaction cup (9);
the manipulator and transit module (5) carries the reaction cup (9) into the incubation module (4), to incubate the reaction cup (9); and
the reaction cup (9) in the incubation module (4) is carried by the manipulator and transit module (5) to the magnetic-separation cleaning module (6) for cleaning; and the cleaned reaction cup (9) is carried by the manipulator and transit module (5) to the detection module (7) for detection,
**characterized in that**
the manipulator and transit module (5) comprises a first manipulator (501), a second manipulator (502), and a transit device (503); the first manipulator (501) is used for carrying the reaction cup (9) in the incubation module (4) to the magnetic-separation cleaning module (6) for cleaning, and carrying it again to the transit device (503); and the second manipulator (502) is used for carrying the reaction cup (9) on the transit device (503) to the detection module (7) for detection,
wherein the first manipulator (501) and the second manipulator (502) both have a gripping jaw (50101, 50201) used for carrying the reaction cup (9); the gripping jaw (50101, 50201) are each disposed on a gripping jaw retractable rack (50102, 50202) and the gripping jaw retractable rack (50102, 50202) is controlled by a retractable motion motor (50103, 50203); a slider (50115, 50204) is connected to the retractable motion motor (50103, 50203), and is driven by an up-and-down motion motor (50105, 50205) to drive the gripping jaw (50101, 50201) to move up and down; and a rotation shaft (50106, 50206) is further connected to the retractable motion motor (50103, 50203) and is driven by a rotary motion motor (50107, 50207) to rotate, thus driving the gripping jaw (50101, 50201) to rotate,
and wherein the first manipulator (501) is further provided with an operating base (50108); a first-manipulator horizontal motion screw rod (50109) is sleeved on the operating base (50108), and is driven by a first-manipulator horizontal motion motor (50110), such that the first manipulator (501) is able to move back and forth along a first-manipulator horizontal motion guide rail (50111).

2. The fully-automatic chemiluminescence immunoassay analyzer according to claim 1, wherein the transit device comprises a transit base (50301) which is provided with several cup hole sites for accommodating the reaction cups, and a transit rotary motor is disposed below the transit base (50301) and used for driving the transit base (50301) to make a corresponding rotary motion.

3. The fully-automatic chemiluminescence immunoassay analyzer according to claim 1, wherein the robot-arm sample injection module (3) comprises a first sampling device (301) disposed with a sampling needle assembly (30101) and a second sampling device (302) disposed with a pipette tip assembly (30201); the first sampling device (301) is used for drawing the reaction reagent and adding it to the reaction cup (9), and the second sampling device is used for drawing the sample and adding it to the reaction cup (9); and the robot-arm sample injection module (3) further comprises a transverse motion assembly (303), a longitudinal motion assembly (304), and an up-and-down motion assembly (305), wherein the transverse motion assembly (303) drives the first sampling device (301) and the second sampling device (302) separately to make a transverse motion, the longitudinal motion assembly (304) drives the first sampling device (301) and the second sampling device (302) separately to make a longitudinal motion, and the up-and-down motion assembly (305) drives the first sampling device (301) and the second sampling device (302) separately to make an up-and-down motion.

4. The fully-automatic chemiluminescence immunoassay analyzer according to claim 3, wherein the transverse motion assembly (303) comprises a first transverse motion motor (30301) which drives a first transmission shaft (30302) to rotate, and drives the first sampling device (301) via the first transmission shaft (30302) to move transversely on a first transverse motion shaft (30303); and the transverse motion assembly (303) further comprises a second transverse motion motor (30304) which drives a second transmission shaft (30305) to rotate and drives the second sampling device (302) via the second transmission shaft (30305) to move transversely on a second transverse motion shaft (30306).

5. The fully-automatic chemiluminescence immunoassay analyzer according to claim 4, wherein the longitudinal motion assembly (304) comprises a first longitudinal motion motor (30401) which drives the first sampling device (301) to move longitudinally along a first longitudinal motion shaft (30402) and a second longitudinal motion shaft (30403); and the longitudinal motion assembly (304) further comprises a second longitudinal motion motor (30404) which drives the second sampling device (302) to move longitudinally along a third longitudinal motion shaft (30405) and a fourth longitudinal motion shaft (30406).

6. The fully-automatic chemiluminescence immunoassay analyzer according to claim 5, wherein the up-and-down motion assembly (305) comprises a first up-and-down motion motor (30501), the sampling needle assembly (30101) and a first up-and-down motion slider (30502) are connected, the first up-and-down motion slider (30502) is sleeved on a first up-and-down motion screw rod (30503), the first up-and-down motion motor (30501) drives the first up-and-down motion screw rod (30503) to move, and the first up-and-down motion slider (30502) on the first up-and-down motion screw rod (30503) drives the sampling needle assembly (30101) to move up and down along a first guide rail (30504); the up-and-down motion assembly (305) further comprises a second up-and-down motion motor (30505), the pipette tip assembly (30201) and a second up-and-down motion slider (30506) are connected, the second up-and-down motion slider (30506) is sleeved on a second up-and-down motion screw rod (30507), the second up-and-down motion motor drives (30505) the second up-and-down motion screw rod (30507) to move, and the second up-and-down motion slider (30506) on the second up-and-down motion screw rod (30507) drives the pipette tip assembly (30201) to move up and down along a second guide rail (30508).

7. The fully-automatic chemiluminescence immunoassay analyzer according to claim 1, wherein the reaction cup conveyance module (2) comprises a reaction cup conveyance chamber (201), and the reaction cup (9) falling from the reaction cup conveyance chamber (201) is adjusted in posture by means of a reaction cup flipper (202); and the reaction cup conveyance module (2) further comprises a reaction cup carrier (203) which pushes the reaction cup (9) after posture adjustment to a designated position, wherein a moving track of the reaction cup carrier (203) is below the incubation module (4).

8. The fully-automatic chemiluminescence immunoassay analyzer according to claim 7, wherein the reaction cup (9) slides in along a reaction cup cassette holder (204), and a reaction cup transfer bracket (205) is located on the bottom portion of the reaction cup conveyance chamber (201); a reaction cup transfer groove (20501) that fits the reaction cup cassette holder (204) is disposed on the reaction cup transfer bracket (205), and the reaction cup transfer bracket (205) moves back and forth with the synergy of a cup transfer fitting groove (20502); and the reaction cup (9) in the reaction cup conveyance chamber (201) enters the reaction cup transfer groove (20501) and then falls into the reaction cup flipper (202).

9. The fully-automatic chemiluminescence immunoassay analyzer according to claim 8, wherein the robot-arm sample injection module (3) adds the reaction reagent and the sample separately to the reaction cup (9) which is pushed by the reaction cup carrier (203) to the designated position, and then the manipulator and transit module (5) carries the reaction cup (9) into an incubation base (401) of the incubation module (4); and the incubation base (401) is secured on an incubation base vertical panel (402), wherein an incubation sheet (404) is disposed on the bottom of the incubation base (401) and insulating sheets (404) are respectively disposed at the two sides of the incubation base (401).

10. The fully-automatic chemiluminescence immunoassay analyzer according to claim 1, wherein the magnetic-separation cleaning module (6) comprises a magnetic-separation cleaning needle (601), and a rotatable magnetic-separation seat (602) is disposed below the magnetic-separation cleaning needle (601), the magnetic-separation seat (602) having at least one reaction cup hole (60201); and the magnetic-separation cleaning module (6) further comprises a magnetic cleaning pool (603), wherein the magnetic cleaning pool (603) comprises a cleaning solution path (60301) and a cleaning needle liquid discharge path (60302), a cleaning solution enters through a cleaning solution inlet (604) in the cleaning solution path (60301) and then goes into the reaction cup (9) through a cleaning solution pointed outlet (605) for cleaning; and the magnetic-separation cleaning needle (601) is used for discharging the waste liquid in the reaction cup (9) after cleaning through the cleaning needle liquid discharge path (60302).

11. The fully-automatic chemiluminescence immunoassay analyzer according to claim 10, wherein an up-and-down motion of the magnetic-separation cleaning needle (601) is controlled by a magnetic-cleaning lifting motor (60708); the magnetic-separation cleaning module (6) is further disposed with a lifting limit structure (607); the lifting limit structure (607) comprises a magnetic-cleaning lifting upper plate (60701) and a magnetic-cleaning lifting lower plate (60702), wherein a limit optocoupler (60703), a cleaning needle lifting stopper (60704), a lifting screw rod (60705), and a guide rod (60706) are disposed between the magnetic-cleaning lifting upper plate (60701) and the magnetic-cleaning lifting lower plate (60702); a slider (60707) is slidably connected on the lifting screw rod (60705), the magnetic-cleaning lifting motor (60708) drives the lifting screw rod (60705) to rotate, the slider (60707) is connected to the guide rod (60706) and the magnetic-separation cleaning needle (601), and the magnetic-separation cleaning needle (601) is driven by the slider (60707) to move up and down along the guide rod (60706).

12. The fully-automatic chemiluminescence immunoassay analyzer according to claim 11, wherein the magnetic-separation seat (602) is driven by a magnetic-separation seat rotary motor (608) to rotate, and a rotation limit structure (609) is disposed below the magnetic-separation seat rotary motor (608); and a magnet (60203) is disposed inside the magnetic-separation seat (602).

## Patentansprüche

1. Vollautomatischer Chemilumineszenz-Immunotestanalysator, einen Boden (1) umfassend, wobei ein Reaktionsgefäßtransportmodul (2), ein Roboterarmprobeninjektionsmodul (3), ein Inkubationsmodul (4), ein Manipulator- und Durchfuhrmodul (5), ein Magnetabscheidung-Reinigungsmodul (6) und ein Erfassungsmodul (7) unabhängig voneinander auf dem Boden (1) integriert sind, wobei
das Reaktionsgefäßtransportmodul (2) zum Transport eines Reaktionsgefässes (9) zu einer vorbestimmten Position benutzt wird, sodass das Roboterarmprobeninjektionsmodul (3) in der Lage ist, ein Reaktionsreagenz und eine Probe getrennt dem Reaktionsgefäß (9) hinzuzufügen;
das Manipulator- und Durchfuhrmodul (5) das Reaktionsgefäß (9) in das Inkubationsmodul (4) zur Inkubation des Reaktionsgefässes (9) trägt; und
das Reaktionsgefäß (9) in dem Inkubationsmodul (4) von dem Manipulator- und Durchfuhrmodul (5) zum Magnetabscheidung-Reinigungsmodul (6) zur Reinigung getragen wird; und das gereinigte Reaktionsgefäß (9) vom Manipulator- und Durchfuhrmodul (5) zum Erfassungsmodul (7) zur Erfassung getragen wird,
**dadurch gekennzeichnet, dass** das Manipulator- und Durchfuhrmodul (5) einen ersten Manipulator (501), einen zweiten Manipulator (502), und eine Durchfuhrvorrichtung (503) umfasst; der erste Manipulator (501) zur Beförderung des Reaktionsgefässes (9) in das Inkubationsmodul (4) zum Magnetabscheidung-Reinigungsmodul (6) zur Reinigung, und zur erneuten Beförderung dieses zur Durchfuhrvorrichtung (503) benutzt wird; und der zweite Manipulator (502) zur Beförderung des Reaktionsgefässes (9) auf der Durchfuhrvorrichtung (503) zur Erfassungsmodul (7) zur Erfassung benutzt wird,
wobei der erste Manipulator (501) und der zweite Manipulator (502) beide eine Klemmbacke (50101, 50102) aufweisen, die zur Beförderung des Reaktionsgefässes (9) benutzt wird; die Klemmbacken jede auf einem zurückziehbaren Klemmbackenrahmen (50102, 50202) angeordnet sind; und der zurückziehbaren Klemmbackenrahmen (50102, 50202) von einem zurückziehbaren Bewegungsmotor (50103, 50203) gesteuert ist; ein Schieber (50115, 50204) mit dem zurückziehbaren Bewegungsmotor (50103, 50203) verbunden ist, und von einem Auf- und Abwärtsbewegungsmotor (50105, 50205) zum Antrieb der Klemmbacke (50101, 50102) zur einer Auf- und Abwärtsbewegung angetrieben ist; und eine Drehwelle (50106, 50106) weiter mit dem zurückziehbaren Bewegungsmotor (50103, 50203) verbunden ist und zur Drehung von einem Drehbewegungsmotor (50107, 50207) angetrieben ist, wodurch die Klemmbacke (50101, 50201) zur Drehung angetrieben ist, und wobei der erste Manipulator (501) weiter mit einem Betriebsboden (50108) versehen ist; eine Horizontalbewegungsgewindestange (50109) des ersten Manipulators auf dem Betriebsboden (50108) umhüllt ist, und von dem Horizontalbewegungsmotor (50110) des ersten Manipulators angetrieben ist sodass der erste Manipulator (501) hin und her eine Horizontalbewegungsführungsbahn (50111) des ersten Manipulators entlang bewegen kann.

2. Vollautomatischer Chemilumineszenz-Immunotestanalysator nach Anspruch 1, wobei die Durchfuhrvorrichtung einen Durchfuhrboden (50301) umfasst, der mit mehreren Gefäßlöcherstellen zum Unterbringen der Reaktionsgefäßen versehen ist, und ein Durchfuhrdrehmotor (50301) unter dem Durchfuhrboden (50301) angeordnet ist, und zum Antrieb des Durchfuhrbodens (50301) für eine entsprechende Drehbewegung benutzt wird.

3. Vollautomatischer Chemilumineszenz-Immunotestanalysator nach Anspruch 1, wobei das Roboterarmprobeninjektionsmodul (3) eine erste Probenahmevorrichtung (301) mit einem Probenahmenadelaufbau (30101) angeordnet und eine zweite Probenahmevorrichtung (302) mit einem Pipettenspitzenaufbau (30201) angeordnet umfasst; die erste Probenahmevorrichtung (301) zum Saugen des Reaktionsreagenzes und zur Hinzufügung dieses zum Reaktionsgefäß (9) benutzt wird, und die zweite Probenahmevorrichtung (302) zum Saugen der Probe und zur Hinzufügung dieser zum Reaktionsgefäß (9) benutzt wird; und das Roboterarmprobeninjektionsmodul (3) weiter einen Querbewegungsaufbau (303), einen Längsbewegungsaufbau (304), und einen Aufbau für Auf- und Abwärtsbewegung (305) umfasst, wobei der Querbewegungsaufbau (303) die erste Probenahmevorrichtung (301) und die zweite Probenahmevorrichtung (302) getrennt zu einer Querbewegung antreibt, der Längsbewegungsaufbau (304) die erste Probenahmevorrichtung (301) und die zweite Probenahmevorrichtung (302) getrennt zu einer Längsbewegung antreibt, und der Aufbau für Auf- und Abwärtsbewegung (305) die erste Probenahmevorrichtung (301) und die zweite Probenahmevorrichtung (302) getrennt zu einer Auf- und Abwärtsbewegung antreibt.

4. Vollautomatischer Chemilumineszenz-Immunotestanalysator nach Anspruch 3, wobei der Querbewegungsaufbau (303) einen ersten Querbewegungsmotor (30301) umfasst, der eine erste Übertragungswelle (30302) zum Drehen antreibt, und die erste Probenahmevorrichtung (301) über die erste Übertragungswelle (30302) zu einer Querbewegung auf eine erste Querbewegungswelle (30303) antreibt; und der Querbewegungsaufbau (303) weiter einen zweiten Querbewegungsmotor (30304) umfasst, der eine zweite Übertragungswelle (30305) zum Drehen antreibt, und die zweite Probenahmevorrichtung (302) über die zweite Übertragungswelle (30305) zu einer Querbewegung auf eine zweite Querbewegungswelle (30306) antreibt.

5. Vollautomatischer Chemilumineszenz-Immunotestanalysator nach Anspruch 4, wobei der Längsbewegungsaufbau (304) einen ersten Längsbewegungsmotor (30401) umfasst, der die erste Probenahmevorrichtung (301) zu einer Längsbewegung eine erste Längsbewegungswelle (30402) und eine zweite Längsbewegungswelle (30403) entlang antreibt; und der Längsbewegungsaufbau (304) weiter einen zweiten Längsbewegungsmotor (30404) umfasst, der die zweite Probenahmevorrichtung (302) zu einer Längsbewegung eine dritte Längsbewegungswelle (30405) und eine vierte Längsbewegungswelle (30406) entlang antreibt.

6. Vollautomatischer Chemilumineszenz-Immunotestanalysator nach Anspruch 5, wobei der Aufbau für Auf- und Abwärtsbewegung (305) einen ersten Motor für Auf- und Abwärtsbewegung (30501) umfasst, der Probennadelaufbau (30101) und ein erster Schieber für Auf- und Abwärtsbewegung (30502) verbunden sind, der erste Schieber für Auf- und Abwärtsbewegung (30502) eine Gewindestange für Auf- und Abwärtsbewegung (30503) umhüllt, der erste Motor für Auf- und Abwärtsbewegung (30501) die erste Gewindestange für Auf- und Abwärtsbewegung (30503) zum Bewegen antreibt, und der Schieber für Auf-und Abwärtsbewegung (30502) auf der ersten Gewindestange für Auf- und Abwärtsbewegung (30503) den ersten Probenahmenadelaufbau (30101) zur einer Auf- und Abwärtsbewegung antreibt eine erste Führungsbahn (30504) entlang antreibt; der Aufbau für Auf- und Abwärtsbewegung (305) weiter einen zweiten Motor für Auf- und Abwärtsbewegung (30505) umfasst, der Pipettenspitzenaufbau (30201) und ein zweiter Auf-und-Abwärtsbewegungsschieber (30506) verbunden sind, der zweite Auf-und-Abwärtsbewegungsschieber (30506) eine zweite Auf-und-Abwärtsgewindestange (30507) umhüllt, der zweite Auf-und-Abwärtsbewegungsmotor (30505) die zweite Auf-und-Abwärtsgewindestange (30507) zur Bewegung antreibt, und der zweite Auf-und-Abwärtsbewegungsschieber (30506) auf der zweiten Auf-und-Abwärtsgewindestange (30507) den Pipettenspitzenaufbau (30201) zur Auf- und Abwärtsbewegung eine zweite Führungsbahn antreibt (30508) entlang antreibt.

7. Vollautomatischer Chemilumineszenz-Immunotestanalysator nach Anspruch 1, wobei das Reaktionsgefäßtransportmodul (2) eine Reaktionsgefäßtransportkammer (201) umfasst, und das Reaktionsgefäß (9) von der Reaktionsgefäßtransportkammer (201) abfallend in Lage mittels einer Reaktionsgefäßkippvorrichtung (202) eingestellt wird; und das Reaktionsgefäßtransportmodul (2) weiter einen Reaktionsgefäßträger (203) umfasst, der auf das Reaktionsgefäß (9) nach der Lageeinstellung zu einer bestimmten Position drückt, wobei eine bewegliche Spur des Reaktionsgefäßträgers (203) unter dem Inkubationsmodul (4) liegt.

8. Vollautomatischer Chemilumineszenz-Immunotestanalysator nach Anspruch 7, wobei das Reaktionsgefäß (9) einen Reaktionsgefäßkassettenhalter (204) entlang einschiebt, und eine Reaktionsgefäßübertragungsklammer (205) auf dem Unterteil der Reaktionsgefäßtransportkammer (201) gelegen ist; eine Reaktionsgefäßübertragungsnut (20501), die dem Reaktionsgefäßkassettenhalter (204) angepasst auf der Reaktionsgefäßübertragungsklammer (205) angeordnet ist, und die Reaktionsgefäßübertragungsklammer (205) hin und her mit der Synergie einer Reaktionsgefäßübertragungsbeschlagsnut (20502) bewegt; und das Reaktionsgefäß (9) in der Reaktionsgefäßtransportkammer (201) die Reaktionsgefäßübertragungsnut (20501) eintritt and dann in die Reaktionsgefäßkippvorrichtung (202) fällt.

9. Vollautomatischer Chemilumineszenz-Immunotestanalysator nach Anspruch 8, wobei das Roboterarmprobeninjektionsmodul (3) das Reaktionsreagenz und die Probe getrennt dem Reaktionsgefäß (9) hinzufügt, das von dem Reaktionsgefäßträger (203) zu dem bestimmten Position gedrückt wird, und dann das Manipulator- und Durchfuhrmodul (5) das Reaktionsgefäß (9) in einen Inkubationsboden (401) des Inkubationsmoduls (4) drückt; und der Inkubationsboden (401) auf einem Inkubationsbodenvertikalplatte (402) befestigt ist, wobei eine Inkubationsplatte (404) auf der Unterseite des Inkubationsbodens (401) angeordnet ist und Isolationsplatten (404) jeweils auf den beiden Seiten des Inkubationsbodens (401) angeordnet sind.

10. Vollautomatischer Chemilumineszenz-Immunotestanalysator nach Anspruch 1, wobei das Magnetabscheidung-Reinigungsmodul (6) eine Magnetabscheidung-Reinigungsnadel (601) umfasst, und ein drehbarer Magnetabscheidungssitz (602) unter der Magnetabscheidung-Reinigungsnadel (601) angeordnet ist, wobei der Magnetabscheidungssitz (602) zu mindesten ein Reaktionsgefäßloch aufweist (60201); und der Magnetabscheidungssitz (602) (6) weiter ein Magnetreinigungsbecken (603) umfasst, wobei das Magnetreinigungsbecken (603) einen Reinigungslösungsweg (60301) und einen Reinigungsnadelflüssigkeitsablassweg (60302) umfasst, eine Reinigungslösung über den Reinigungslösungseinlass (604) in den Reinigungslösungsweg (60301) eindringt, und dann über einen Reinigungslösungsspitzenauslass (605) in das Reaktionsgefäß (9) zur Reinigung übergeht; die Magnetabscheidung-Reinigungsnadel (601) zum Ablass des flüssigen Abfalls in das Reaktionsgefäß (9) über den Reinigungsnadelflüssigkeitsablassweg (60302) nach der Reinigung benutzt wird.

11. Vollautomatischer Chemilumineszenz-Immunotestanalysator nach Anspruch 10, wobei eine Auf-und-Abwärtsbewegung der Magnetabscheidung-Reinigungsnadel (601) von einem Magnetreinigungshubmotor (60708) gesteuert wird; das Magnetabscheidung-Reinigungsmodul (6) weiter mit einer Hubgrenzwertstruktur (607) angeordnet ist; die Hubgrenzwertstruktur (607) eine Magnetreinigungshuboberplatte (60701) und eine Magnetreinigungshubunterplatte (60702) umfasst, wobei ein Grenzwert-Optokoppler (60703), ein Magnetreinigungshubstopper (60704), eine Hubgewindestange (60705), und eine Führungsstange (60706) zwischen der Magnetreinigungshuboberplatte (60701) und der Magnetreinigungshubunterplatte (60702) angeordnet sind; ein Schieber (60707) verschiebbar auf der Hubgewindestange (60705) verbunden ist, der Magnetreinigungshubmotor (60708) die Hubgewindestange (60705) zum Drehen antreibt, der Schieber (60707) mit der Führungsstange (60706) und mit der Magnetabscheidung-Reinigungsnadel (601) verbunden ist, und die Magnetabscheidung-Reinigungsnadel (601) von dem Schieber (60707) zu einer Auf-und-Abwärtsbewegung die Führungsstange (60706) entlang angetrieben ist.

12. Vollautomatischer Chemilumineszenz-Immunotestanalysator nach Anspruch 11, wobei der Magnetabscheidungssitz (602) von einem Magnetabscheidungssitzdrehmotor (608) zum Drehen angetrieben ist, und eine Drehgrenzwertstruktur (609) unter dem Magnetabscheidungssitzdrehmotor (608) angeordnet ist; und ein Magnet (60203) innerhalb des Magnetabscheidungssitzes (602) angeordnet ist.

## Revendications

1. Analyseur pour essai immunologique par chimioluminescence entièrement automatique comprenant une embase (1), où un module de transfert de coupelle de réaction (2), un module d'injection d'échantillon à bras robotique (3), un module d'incubation (4), un module de transit avec manipulateurs (5), un module de nettoyage par séparation magnétique (6), et un module de détection (7) sont intégrés en étant mutuellement indépendants sur l'embase (1), analyseur dans lequel
le module de transfert de coupelle de réaction (2) est utilisé pour transférer une coupelle de réaction (9) vers une position prédéterminée de sorte que le module d'injection d'échantillon à bras robotique (3) soit apte à ajouter séparément un réactif réactionnel et un échantillon dans la coupelle de réaction (9) ;
le module de transit avec manipulateurs (5) transporte la coupelle de réaction (9) dans le module d'incubation (4), afin d'incuber la coupelle de réaction (9) ;
et la coupelle de réaction (9) dans le module d'incubation (4) est transportée par le module de transit avec manipulateurs (5) vers le module de nettoyage par séparation magnétique (6) aux fins de nettoyage ; et la coupelle de réaction nettoyée (9) est transportée par le module de transit avec manipulateurs (5) vers le module de détection à des fins de détection,
**caractérisé en ce que** le module de transit avec manipulateurs (5) comprend un premier manipulateur (501), un deuxième manipulateur et un dispositif de transit (503) ; le premier manipulateur (501) est utilisé pour transporter la coupelle de réaction (9) dans le module d'incubation (4), vers le module de nettoyage par séparation magnétique (6) à des fins de nettoyage, et pour la transporter à nouveau vers le dispositif de transit (503) ; et le deuxième manipulateur est utilisé pour transporter la coupe de réaction (9) sur le dispositif de transit (503) vers le module de détection (7) à des fins de détection, où le premier manipulateur (501) et le deuxième manipulateur ont tous les deux une mâchoire de préhension (50101, 50201) utilisée pour transporter la coupelle de réaction (9) ; les mâchoires de préhension (50101, 50201) sont chacune disposées sur un support rétractable (50102, 50202) pour mâchoire de préhension et le support rétractable pour mâchoire de préhension (50102, 50202) est commandé par un moteur de mouvement rétractable (50103, 50203) ; un coulisseau (50115, 50204) est raccordé au moteur de mouvement rétractable (50103, 50203), et est entraîné par un moteur de mouvement vers de haut en bas (50105, 50205) afin d'entraîner la mâchoire de préhension (50101, 50201) à se mouvoir de haut en bas ; et un arbre de rotation (50106, 50206) est en outre raccordé au moteur de mouvement rétractable (50103, 50203) et est entraîné à rotation par un moteur de mouvement rotatif (50107, 50207), entraînant donc la mâchoire de préhension à tourner, et où le premier manipulateur (501) est en outre muni d'une embase d'opération (50108) ; une tige filetée (50109) pour mouvement horizontal du premier manipulateur est gainée sur l'embase d'opération (50108) et est entraînée par un moteur (50110) à mouvement horizontal de premier manipulateur de sorte que le premier manipulateur est apte à se mouvoir en va-et-vient le long d'un rail de guidage (50111) pour mouvement horizontal du premier manipulateur.

2. Analyseur pour essai immunologique par chimioluminescence entièrement automatique selon la revendication 1, dans lequel le dispositif de transit comprend une embase de transit (50301), qui est pourvue de plusieurs endroits d'orifice pour coupelles aptes à loger les coupelles de réaction, et un moteur rotatif de transit est disposé au-dessous de l'embase de transit (50301) et est utilisé pour entraîner l'embase de transit (50301) à faire un mouvement de rotation correspondant.

3. Analyseur pour essai immunologique par chimioluminescence entièrement automatique selon la revendication 1, dans lequel le module d'injection d'échantillon à bras robotique (3) comprend un premier dispositif d'échantillonnage (301) disposé avec un assemblage d'aiguille d'échantillonnage (30101) et un deuxième dispositif d'échantillonnage (302) disposé avec un assemblage à bout de pipette (30201) ; le premier dispositif d'échantillonnage (301) est utilisé pour prélever le réactif réactionnel et pour l'ajouter à la coupelle de réaction (9), et le deuxième dispositif d'échantillonnage (302) est utilisé pour prélever l'échantillon et pour l'ajouter à la coupe de réaction (9) ; et le module d'injection d'échantillon à bras robotique (3) comprend en outre un assemblage pour mouvement transversal (303), un assemblage pour mouvement longitudinal (304), et un assemblage pour mouvement de haut en bas (305), où l'assemblage pour mouvement transversal (303) entraîne séparément le premier dispositif d'échantillonnage (301) et le deuxième dispositif d'échantillonnage (302) de façon à ce qu'ils fassent un mouvement transversal, l'assemblage pour mouvement longitudinal (304) entraîne séparément le premier dispositif d'échantillonnage (301) et le deuxième dispositif d'échantillonnage (302) de façon à ce qu'ils fassent un mouvement longitudinal et l'assemblage pour mouvement de haut en bas (305) entraîne séparément le premier dispositif d'échantillonnage (301) et le deuxième dispositif d'échantillonnage (302) de façon à ce qu'ils fassent un mouvement de haut en bas.

4. Analyseur pour essai immunologique par chimioluminescence entièrement automatique selon la revendication 3, dans lequel l'assemblage pour mouvement transversal (303) comprend un premier moteur de mouvement transversal (30301) qui entraîne à rotation un premier arbre de transmission (30302), et entraîne le premier dispositif d'échantillonnage (301) par l'intermédiaire du premier arbre de transmission (30302) à se mouvoir transversalement sur un premier arbre de mouvement transversal (30303) ; et l'assemblage pour mouvement transversal (303) comprend en outre un deuxième moteur de mouvement transversal (30304) qui entraîne à rotation un deuxième arbre de transmission (30305), et entraîne le deuxième dispositif d'échantillonnage (301) par l'intermédiaire du deuxième arbre de transmission (30305) à se mouvoir transversalement sur un deuxième arbre de mouvement transversal (30306).

5. Analyseur pour essai immunologique par chimioluminescence entièrement automatique selon la revendication 4, dans lequel l'assemblage pour mouvement longitudinal (304) comprend un premier moteur de mouvement longitudinal (30401) qui entraîne le premier dispositif d'échantillonnage (301) à se mouvoir longitudinalement le long d'un premier arbre de mouvement longitudinal (30402) et d'un deuxième arbre de mouvement longitudinal (30403) ; et l'assemblage pour mouvement longitudinal (304) comprend en outre un deuxième moteur de mouvement longitudinal (30304) qui entraîne le deuxième dispositif d'échantillonnage (302) à se mouvoir longitudinalement le long d'un troisième arbre de mouvement longitudinal (30405) et d'un quatrième arbre de mouvement longitudinal (30406).

6. Analyseur pour essai immunologique par chimioluminescence entièrement automatique selon la revendication 5, dans lequel l'assemblage pour mouvement de haut en bas (305) comprend un premier moteur de mouvement de haut en bas (30501), l'assemblage d'aiguille d'échantillonnage (30101) et un premier coulisseau de mouvement de haut en bas (30502) sont raccordés, et le premier coulisseau de mouvement de haut en bas (30502) est gainé sur une première tige filetée de mouvement de haut en bas (30503), le premier moteur de mouvement de haut en bas (30501) entraîne la première tige filetée de mouvement de haut en bas (30503) de façon à ce qu'elle se déplace, et le premier coulisseau de mouvement de haut en bas (30502) sur la première tige filetée de mouvement de haut en bas (30503) entraîne l'assemblage d'aiguille d'échantillonnage (30101) de façon à ce qu'il se déplace de haut en bas le long d'un premier rail de guidage (30504) ; l'assemblage pour mouvement de haut en bas (305) comprend en outre un deuxième moteur de mouvement de haut en bas (30505), l'assemblage à bout de pipette (30201) et un deuxième coulisseau de mouvement de haut en bas (30506) sont raccordés, le deuxième coulisseau de mouvement de haut en bas (30506) est gainé sur une deuxième tige filetée de mouvement de haut en bas (30507), le deuxième moteur de mouvement de haut en bas (30505) entraîne la deuxième tige filetée de mouvement de haut en bas (30507), et le deuxième coulisseau de mouvement de haut en bas (30506) sur la deuxième tige filetée de mouvement de haut en bas (30507) entraîne l'assemblage à bout de pipette à se mouvoir de haut en bas le long d'un deuxième rail de guidage (30508).

7. Analyseur pour essai immunologique par chimioluminescence entièrement automatique selon la revendication 1, dans lequel le module de transfert de coupelle de réaction (2) comprend une chambre de transfert de coupelle de réaction (201), et la coupelle de réaction (9) tombant de la chambre de transfert de coupelle de réaction (201) est ajustée en position au moyen d'un dispositif basculant de coupelle de réaction (202) ; et le module de transfert de coupelle de réaction (2) comprend en outre un transporteur de coupelle de réaction (203) qui pousse la coupelle de réaction (9) après l'ajustement de position vers une position désignée, où une voie de mouvement du transporteur de coupelle de réaction (203) se trouve au-dessous du module d'incubation (4).

8. Analyseur pour essai immunologique par chimioluminescence entièrement automatique selon la revendication 7, dans lequel la coupelle de réaction (9) coulisse le long d'un porte-cassette de coupelle de réaction (204), et un support de transfert de coupelle de réaction (205) est situé sur la partie inférieure de la chambre de transfert de coupelle de réaction (201) ; une rainure de transfert de coupelle de réaction (20501) qui s'adapte au porte-cassette de coupelle de réaction (204) est disposée sur le support de transfert de coupelle de réaction (205), et le support de transfert de coupelle de réaction (205) se déplace en va et vient en synergie avec une rainure de transfert de coupelle de réaction (20502) ; et la coupelle de réaction (9) dans la chambre de transfert de coupelle de réaction (201) pénètre dans la rainure de transfert de coupelle de réaction (20501) et tombe ensuite dans le dispositif basculant de coupelle de réaction (202).

9. Analyseur pour essai immunologique par chimioluminescence entièrement automatique selon la revendication 8, dans lequel le module d'injection d'échantillon à bras robotique (3) ajoute séparément le réactif réactionnel et l'échantillon à la coupelle de réaction (9) qui est poussée par le transporteur de coupelle de réaction (203) vers la position désignée, ensuite le module de transit avec manipulateur (5) transporte la coupelle de réaction (9) dans une embase d'incubation (401) du module d'incubation (4) ; et l'embase d'incubation (401) est fixée sur un panneau vertical d'embase d'incubation (402), où une plaque d'incubation (404) est disposée sur le fond de l'embase d'incubation (401) et des plaques isolantes (404) sont disposées respectivement sur les deux côtés de l'embase d'incubation (401).

10. Analyseur pour essai immunologique par chimioluminescence entièrement automatique selon la revendication 1, dans lequel le module de nettoyage par séparation magnétique (6) comprend une aiguille de nettoyage par séparation magnétique (601), et un siège rotatif de séparation magnétique (602) est disposé au-dessous de l'aiguille de nettoyage par séparation magnétique (601), le siège rotatif de séparation magnétique (602) présentant au moins un orifice de coupelle de réaction (60201) ; et le module de nettoyage par séparation magnétique (6) comprend en outre un bassin de nettoyage magnétique (603), où le bassin de nettoyage magnétique (603) comprend une voie de solution de nettoyage (60301) et une voie d'évacuation de liquide d'aiguille de nettoyage (60302), une solution de nettoyage entre par une entrée de solution de nettoyage (604) dans la voie de solution de nettoyage (60301) et ensuite va dans la coupelle de réaction (9) par une sortie pointue de solution de nettoyage (605) à des fins de nettoyage ; et l'aiguille de nettoyage par séparation magnétique (601) est utilisée pour évacuer le liquide résiduel dans la coupelle de réaction (9) après nettoyage par la voie d'évacuation de liquide d'aiguille de nettoyage (60302).

11. Analyseur pour essai immunologique par chimioluminescence entièrement automatique selon la revendication 10, dans lequel un mouvement de haut en bas de l'aiguille de nettoyage par séparation magnétique (601) est commandé par un moteur de levage de nettoyage magnétique (60708) ; le module de de nettoyage par séparation magnétique (6) est en outre disposé avec une structure de limitation de levage (607) ; la structure de limitation de levage (607) comprend une plaque supérieure de levage de nettoyage magnétique (60701) et une plaque inférieure de levage de nettoyage magnétique (60702), où un optocoupleur de limitation (60703), une butée de levage d'aiguille de nettoyage (60704), une tige filetée de levage (60705), et une tige de guidage (60706) sont disposés entre la plaque supérieure de levage de nettoyage magnétique (60701) et la plaque inférieure de levage de nettoyage magnétique (60702) ; un coulisseau (60707) est raccordé de façon à coulisser sur la tige filetée de levage (60705), le moteur de levage de nettoyage magnétique entraîne à rotation la tige filetée de levage (60705), le coulisseau (60707) est raccordé à la tige de guidage (60706) et à l'aiguille de nettoyage par séparation magnétique (601), et l'aiguille de nettoyage par séparation magnétique (601) est entraînée par le coulisseau (60707) de façon à se déplacer de haut en bas le long de la tige de guidage (60706).

12. Analyseur pour essai immunologique par chimioluminescence entièrement automatique selon la revendication 11, dans lequel le siège de séparation magnétique (602) est entraîné à rotation par un moteur rotatif de siège de séparation magnétique (608), et une structure limitatrice de rotation (609) est disposée au-dessous du moteur rotatif de siège de séparation magnétique (608) ; et un aimant (60203) est disposé à l'intérieur du siège de séparation magnétique (602).
